(19) Europäisches Patentamt
European Patent Office
Office européen des brevets

(11) Veröffentlichungsnummer: **0 000 030**
**B1**

(12) **EUROPÄISCHE PATENTSCHRIFT**

(21) Anmeldenummer: **78100050.0**

(22) Anmeldetag: **01.06.78**

(51) Int. Cl.³: **C 07 C 155/02,**
C 07 C 155/08,
A 01 N 47/10

# (54) Schwefelhaltige Diurethane, Verfahren zu deren Herstellung und deren Verwendung als Herbizide

(30) Priorität: **03.06.77 DE 2725146**

(43) Veröffentlichungstag der Anmeldung:
**20.12.78 Patentblatt 78/01**

(45) Bekanntmachung des Hinweises auf die Patenterteilung:
**10.12.80 Patentblatt 80/25**

(84) Benannte Vertragsstaaten:
**BE CH DE FR GB LU NL SE**

(56) Entgegenhaltungen:
**DE - A - 2 617 917**

**CHEMICAL ABSTRACTS, Band 69, 96196s (1968)**

(73) Patentinhaber: **BASF Aktiengesellschaft**
**Carl-Bosch-Strasse 38**
**D - 6700 Ludwigshafen (DE)**

(72) Erfinder: **Schirmer, Ulrich, Dr.**
**Am Goetzenberg 1**
**D - 6900 Heidelberg (DE)**
**Wuerzer, Bruno, Dr.**
**Wilhelm-Busch-Strasse 55**
**D - 6703 Limburgerhof (DE)**
**Rohr, Wolfgang, Dr.**
**Gontardstrasse 4**
**D - 6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 030 B1

# Schwefelhaltige Diurethane, Verfahren zu deren Herstellung und deren Verwendung als Herbizide

Die vorliegende Erfindung betrifft wertvolle schwefelhaltige Diurethane mit ausgezeichneter herbizider Wirkung, Herbizide, die diese Verbindungen enthalten, und Verfahren zur Bekämpfung unerwünschten Pflanzenwachstums mit diesen Verbindungen.

Es ist bekannt, 3 - Methoxycarbonylaminophenyl - N - (3' - methyl - phenyl) carbamat, 3 - Äthoxycarbonylaminophenyl - N - phenylcarbamat, 3 - Methoxycarbonylamino - N - methyl - N - phenylcarbamat (DE-AS 15 67 151) oder 3 - Isopropyl - 2,1,3 - benzothiadiazon - (4) - 2,2 - dioxid (DT-AS 15 42 836) als Herbizide zu verwenden.

Es wurde nun gefunden, daß die neuen Diurethane der allgemeinen Formel

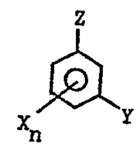

in der Z den Rest

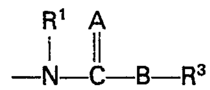

und Y den Rest

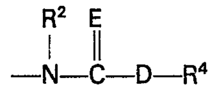

bedeutet, wobei Z immer verschieden ist von Y und $R^1$ und $R^2$ jeweils unabhängig voneinander Wasserstoff, Alkyl mit 1 bis 4 Kohlenstoffatomen (z.B. Methyl, Äthyl, iso-Propyl), Alkoxyalkyl mit 2 bis 4 Kohlenstoffatom (z.B. Methoxymethyl, 2-Methoxyäthyl), Alkoxycarbonylalkyl mit 3 bis 5 Kohlenstoffatomen (z.B. Methoxycarbonylmethyl), Halogenalkyl mit 1 bis 4 Kohlenstoffatomen (z.B. Chlormethyl) oder gegebenenfalls durch Alkyl mit 1 bis 4 Kohlenstoffatomen oder Halogen substituiertes Benzyl, $R^3$ und $R^4$ jeweils unabhängig voneinander unsubstituiertes Alkyl mit 1 bis 6 Kohlenstoffatomen oder durch Halogen oder Alkoxy mit 1 bis 2 Kohlenstoffatomen oder Alkoxycarbonyl oder durch Halogen substituiertes oder unsubstituiertes Phenyl substituiertes Alkyl mit 1 bis 4 Kohlenstoffatomen (z.B. Methyl, Äthyl, 2-Chloräthyl, 2-Methoxyäthyl, Methoxycarbonylmethyl, Benzyl, Isopropyl, n-Propyl, 4-Chlorbenzyl, n-Butyl, sec.-Butyl, tert.-Butyl, iso-Butyl, 2,4-Dichlorbenzyl, 2-Phenyläthyl) oder Alkenyl mit 2 bis 4 Kohlenstoffatomen (z.B. Allyl Buten(1)yl(3)), Alkinyl mit 3 bis Kohlenstoffatomen (z.B. Propargyl, Butin(1)yl(3)), oder gegebenenfalls durch Alkyl mit 1 bis 4 Kohlenstoffatomen substituiertes Cycloalkyl mit 5 bis 8 Kohlenstoffatomen (z.B. Cyclopentyl, Cyclohexyl, 3-Methylcyclohexyl, 2,6-Dimethylcyclohexyl, Cycloheptyl, 4-tert.-Butylcyclohexyl, 3,5-Dimethylcyclohexyl) oder Bicycloalkyl mit 7 bis 8 Kohlenstoffatomen (z.B. Norbonyl) oder Tricycloalkyl mit 10 bis 15 Kohlenstoffatomen (z.B. Tricyclo(4,3,1,$^{2,5}$O$^{1,6}$)-decyl), einen Phenylrest mit ankondensiertem Ringsystem (z.B. Naphthyl oder Indyl), Phenyl oder einen ein- oder mehrfach substituierten Phenylrest, mit den Substituenten Alkyl mit 1 bis 4 Kohlenstoffatomen, Halogen oder Alkoxy mit 1 bis 3 Kohlenstoffatomen, bedeuten, (z.B. Phenyl, 4-Fluorphenyl, 2-Methoxyphenyl, 3-Methylphenyl, 2-Fluorphenyl, 3-Methyl-5-isopropylphenyl, 3-Äthylphenyl, 3-Chlorphenyl, 2,4,6-Trimethylphenyl, 3-Fluorphenyl, 3-Chlor-4-fluorphenyl, 3,4-Dimethylphenyl, 4-Methylphenyl, 3,4-Difluorphenyl, 3-Chlor-4-methylphenyl 3-Bromphenyl, 4-Jodphenyl, 4-Chlorphenyl, 2-Chlorphenyl, 2-Chlor-4-fluorphenyl, 3-Isopropylphenyl, 4-Äthylphenyl, 4-Äthoxyphenyl, 2-Methylphenyl, 3-Methoxyphenyl, 2,6-Dimethylphenyl, 2,4-Dichlorphenyl, 3-Methyl-4-chlorphenyl, 2,4-Dibromophenyl, 4-Methoxyphenyl, 2,3,6-Trimethylphenyl, 3-tert.-Butylphenyl, 3,5-Dichlorphenyl, 2-Methyl-6-äthylphenyl, 2,3-Dimethylphenyl, 2-Methyl-4-chlorphenyl, 2,4,5-Trichlorphenyl, 2,3,5-Trichlorphenyl) und X Wasserstoff, Alkyl mit 1 bis 4 Kohlenstoffatomen (z.B. Methyl), Halogenalkyl mit 1 bis 3 Kohlenstoffatomen (z.B. Trifluormethyl), Alkoxy mit 1 bis 2 Kohlenstoffatomen (z.B. Methoxy), Halogen (z.B. Fluor, Chlor, Brom oder Jod), Nitro oder Amino, n 1 bis 4 und A, B, D, E jeweils unabhängig voneinander Sauerstoff oder Schwefel bedeuten, wobei nicht alle Reste A, B, D, E gleichzeitig Sauerstoff und wobei mindestens einer dieser Reste immer Schwefel bedeutet, eine gute herbizide Wirkung gegen zahlreiche wichtige unerwünschte Pflanzen (z.B. Euphorbia und Chenopodium) und gleichzeitig eine gute Verträglichkeit bei vielen Kulturpflanzen (z.B. Baumwolle und Mais) haben. Diese Wirkungen sind besser als die der bekannten Wirkstoffe.

Die neuen Verbindungen können beispielsweise nach folgenden Verfahren hergestellt werden,

wobei die Reste A, B, D, E, $\bar{R}$, $R^1$, $R^2$, $R^3$, $R^4$, X und n die oben angegebene Bedeutung haben. Wenn im folgenden von Urethanen und Chlorameisensäureestern die Rede ist, sollen unter diesen beiden Sammelbegriffen auch Thiono- Thio- und Dithio-urethane sowie Chlorameisensäurethionoester, Chlorameisensäurethioester und Chlorameisensäuredithioester verstanden werden.

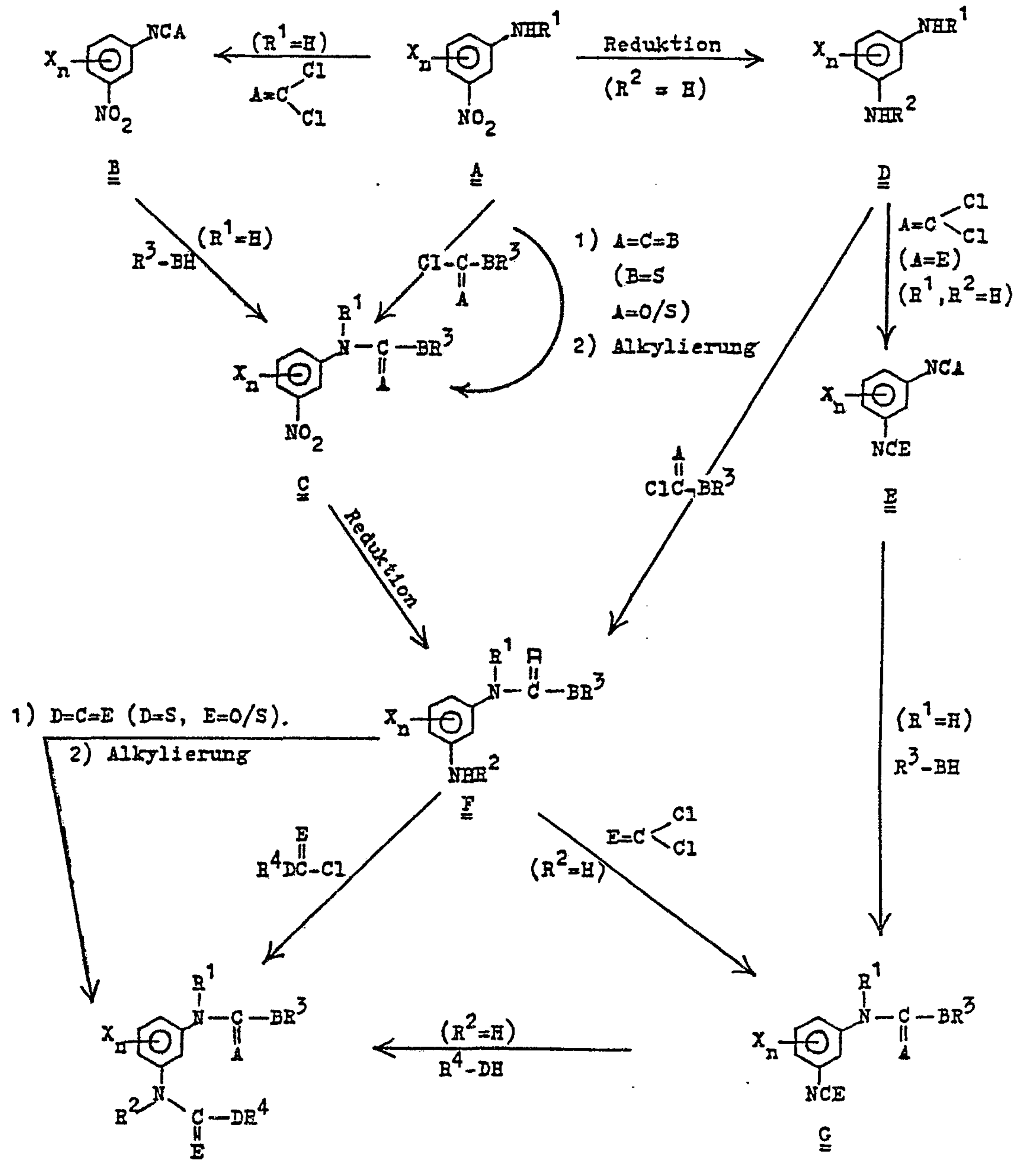


Aus dem aufgezigten Reaktionsschema gehen eindeutig die wechselseitigen Beziehungen zwischen den Ausgangsstoffen hervor. Weiterhin wird deutlich, daß je nach Natur der Substituenten A, B, D, E, $R^1$, $R^2$, $R^3$, $R^4$ und X sowie der Verfügbarkeit der jeweiligen Reaktionspartner der eine oder andere Weg vorteilhaft sein kann. Die Umsetzung von F nach H wird bevorzugt.

Ausgehend von bekannten meta-Nitranilinen (A) lassen sich meta-Nitrophenyliso(thio)cyanate (B) herstellen (W. Siefken, J. Liebigs Annalen der Chemie, *562*, 75 ff. (1949), die ihrerseits mit den Komponenten $R^3$—BH glatt zu den Nitro(thio)urethan (C) reagieren (S. Petersen in Methoden der Organ. Chemie, Band VIII, Seite 131, Georg-Thieme-Verlag, Stuttgart, 4. Auflage, (1952), die allerdings auch direkt aus den meta-Nitranilinen (A) mit Chlorameisensäureestern ($R^3$B—CA—Cl) (DT—OS 16 43 763) oder mit Schwefelkohlenstoff bzw. Kohlenoxysulfid, Base und Alkylierungsmittel (Methoden der Organischen Chemie, Band IX, Seite 831 ff, Georg Thieme-Verlag, Stuttgart, 4. Auflage, 1955) zugänglich

sind. Nachfolgende Reduktion führt zu den Aminoverbindungen (F, $R^2$=H) (S. Schröter in Methoden der Organischen Chemie, Band XI/1, Seite 360 ff., Georg Thieme-Verlag, Stuttgart, 4. Auflage, 1957), die entweder direkt oder nach erfolgter Umwandlung in das am Aminostickstoff monosubstituierte Produkt (F, $R^2$=H) Methoden der Organischen Chemie, Band XI/1, Seite 24 ff., Georg Thieme-Verlag, Stuttgart, 4. Auflage 1957) mit Chlorameisensäureestern ($R^4$D—CE—Cl) (DT-OS 16 43 763) oder mit Schwefelkohlenstoff bzw. Kohlenoxysulfid, Base und Alkylierungsmittel; (Methoden der organischen Chemie, Band IX, Setie 831 ff, Georg-Thieme-Verlag, Stuttgart, 4. Auflage, 1955) zu den gewünschten Diurethanen (H) umgesetzt werden. Die Aminourethane F lassen sich aber auch durch Reaktion von meta-Phenylendiaminen (D) mit Chlorameisensäureestern erhalten. Eine weitere Synthesemöglichkeit besteht in der Reaktion von Aryl-1,3-diiso(thio)-cyanaten (E) mit nur einem Mol Komponente $R^3$—BH, die zu den Iso(thio)cyanatourethanen (G) führt (J. A. Parker, J. J. Thomas und C. L. Zeise, J. org. Chem. *22*, 594 bis 596 (1957)), welche auch durch (Thio)phosgenierung der Aminourethane (F) erhältlich sind (DT-OS 19 14 270, Seite 5, Beispiel 8). Anschließende Reaktion mit der Komponente $R^4$DH führt zu den gewünschten Endprodukten. Grundsätzlich ist zu bemerken, daß die Reihenfolge der —CABR$^3$ bzw. —CEDR$^4$-Gruppierungen beliebig ist.

Nachfolgend werden die bevorzugten Syntheseschritte genauer beschrieben:

a) Die Umsetzung von 3-Nitrophenyl(thio)cyanaten (B) erfolgt mit oder ohne einen für Iso(thio)-cyanatreaktionen gebräuchlichen Katalysator, z.B. tert. Amine (Triäthylamin, 1,4-Diazabicyclo-(2.2.2)-octan), stickstoffhaltige Heterocyclen (Pyridin, 1,2-Dimethylimidazol) oder organische Zinnverbindungen (Dibutylzinndiacetat, Dimethylzinndichlorid) gegebenenfalls in einem unter den Reaktionsbedingungen inerten Lösungsmittel, z.B. Kohlenwasserstoffe (Ligroin, Benzin, Toluol, Pentan, Cyclohexan), Halogenkohlenwasserstoffe (Methylenchlorid, Chloroform, Dichloräthan, Chlorbenzol, o-, m- oder p-Dichlorbenzol), Nitrokohlenwasserstoffe (Nitrobenzol, Nitromethan), Nitrile (Acetonitril, Butyronitril, Benzonitril), Äther (Diäthyläther, Tetrahydrofuran, Dioxan), Ester (Essigsäureäthylester, Propionsäuremethylester), Ketone (Aceton, Methyläthylketon) oder Amide (Dimethylformamid, Formamid) (DT-OS 15 68 138) bei Temperaturen im Bereich von 0 bis 150°C, vorzugsweise in Bereich von 40° bis 100°C.

b) 3-Nitraniline (A) werden mit Chlorameisensäureester in einem geeigneten Lösungsmittel, z.B. Wasser, Alkohole (Methanol, Äthanol, Isopropanol) oder wie under a) angegeben, unter Zuhilfenahme eines üblichen Säurebinders, z.B. Alkalihydroxide, -carbonate, -hydrogencarbonate, Erdalkalioxide, -hydroxide, -carbonate, -hydrogencarbonate, tertiäre organische Basen (z.B. Triäthylamin, Pyridin, N,N-Dimethylanilin, N,N-Dimethylcyclohexylamin, Chinolin, Tributylamin) oder Ausgangsprodukt 3-Nitranilin, bei Temperaturen von −20° bis 150°C, vorzugsweise im Bereich von 20 bis 80°C umgesetzt.

c) die Reduktion der Nitrourethane (C) kann nach einem der bekannten Verfahren durchgeführt werden, beizpielsweise durch katalytische kydrierung, durch ein Metall-Säure-Kombination, z.B. eine Kombination Eisen-Säure, durch eine Metall-Alkohol-Kombination, z.B. Zinkstaub-wäßriger Alkohol, Eisen-wäßriger Alkohol.

d) Für die Umsetzung von m-Phenylendiaminen (D gelten vergleichbare Bedingungen wie für b), wobei es aber vorteilhaft sein kann, mit einem Überschuß an m-Phenylendiaminen zu arbeiten.

e) Die Reaktion der Aminourethane F mit Chlorameisensäureestern erfolgt analog zu b), wobei man auch die z.B. durch katalystische Hydrierung der Nitrourethane C erhaltene Lösung ohne weitere Reinigung direkt einsetzen kann.

Die folgenden Beispiele sollen die Herstellung der neuen Diurethane und ihrer Vorprodukte erläutern:

I. *Nitrourethane*

Beispiel A

Einer Lösung von 64,3 Gewichtsteilen 4-Chlorphenol und 3 Gewichtsteilen Triäthylamin in 430 Gewichtsteilen Toluol (absolut) wurde unter Rühren bei 20 bis 25°C eine Mischung von 85 Gewichtsteilen 3-Nitrophenylisocyanat und 43 Gewichtsteilen Toluol (absolut) zudosiert.

Zur Vervollständigung der Reaktion wurde 1 Stunde bei Raumtemperatur nachgerührt. Nach Kühlen auf 0°C wurde das Reaktionsprodukt abgesaugt:

Fp. 137 bis 138°C

Die Verbindung hat folgende Strukturformel:

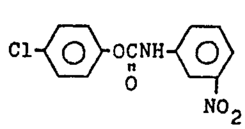

Beispiel B

Zu 138 Gewichtsteilen m-Nitranilin in 500 Gewichtsteilen Tetrahydrofuran (THF) werden 87 Gewichtsteile Natriumhydrogencarbonat gegeben. Unter Rühren tropft man bei Raumtemperatur 120 Gewichtsteile Chlorameisensäurethiomethylester zu, läßt 16 Stunden bei Raumtemperatur nachrühren, filtriert, destilliert das Lösungsmittel am Rotationsverdampfer ab und rührt das erhaltene Öl in Toluol ein. Die sich abscheidenden Kristalle werden abgesaugt und getrocknet:
Fp.: 137—138°C.

Die Verbindung hat folgende Strukturformel:

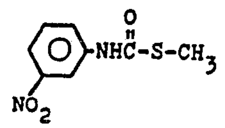

Beispiel C

Zu 26 Gewichtsteilen 3-Nitro-N-methylanilin in 320 Gewichtsteilen Essigsäureäthylester werden 17,4 Gewichtsteile Natriumhydrogencarbonat gegeben. Unter Rühren fügt man langsam 33 Gewichtsteile Chlorameisensäure-m-tolylester zu, läßt 20 Stunden bei Raumtemperatur rühren, filtriert, zieht das Lösungsmittel im Vakuum ab und kristallisiert den verbleibenden Rückstand aus Toluol/Cyclohexan um.
Fp.: 114 bis 116°C.

Die Verbindung hat folgende Strukturformel:

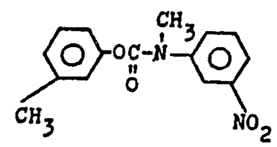

Nach entsprechenden Verfahren können folgende Nitrourethane (C) hergestellt werden:

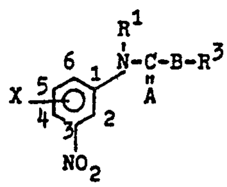

| A | B | X | $R^1$ | $R^3$ | Fp. °C |
|---|---|---|---|---|---|
| S | O | H | H | Methyl | |
| O | O | H | H | 4-Fluorphenyl | 166—167 |
| O | O | H | H | 2,4-Dichlorphenyl | 150—151 |
| O | O | H | H | Methyl | 153—155 |
| O | O | 6-$CH_3$ | H | Methyl | 132—133 |
| O | O | H | Benzyl | 4-Chlorphenyl | |
| O | O | H | H | Phenyl | 123—125 |
| O | O | H | H | 3-Methoxyphenyl | |
| O | O | H | $CH_3$ | Phenyl | 69— 70 |
| O | O | H | H | 2-Fluorphenyl | 145—146 |
| O | O | 6-F | H | Phenyl | 138—140 |
| O | O | H | H | 3-Bromphenyl | 130—131 |

| A | B | X | $R^1$ | $R^3$ | Fp. °C |
|---|---|---|---|---|---|
| O | O | H | $CH_3OCH_2$ | 3-Methylphenyl | |
| O | O | H | H | 3,4-Dimethylphenyl | 130—131 |
| O | O | 5-$CF_3$ | H | Methyl | 86— 87 |
| O | O | H | H | 4-Methoxyphenyl | 132—133 |
| O | O | 6-$CH_3$ | H | Äthyl | 131—133 |
| O | O | H | H | 3-Fluorphenyl | 128—130 |
| O | O | H | H | Äthyl | 64— 66 |
| O | O | 2-$CH_3$ | H | Phenyl | 112—114 |
| O | O | H | H | 2-Chlor-4-fluorphenyl | 146—147 |
| O | O | H | H | 2-Chlorphenyl | 136—138 |
| O | O | 4,6-$F_2$ | H | Methyl | |
| O | O | 4-$CH_3$ | H | Methyl | 114—117 |
| O | O | 2,5-$Cl_2$ | $CH_3$ | 2-Methoxyäthyl | |
| O | O | H | H | 4-Trifluormethylphenyl | |
| O | O | H | H | 4-Äthylphenyl | 86— 88 |
| O | O | 4-Cl | H | Phenyl | 125—127 |
| O | O | H | H | 3-Chlor-4-fluorphenyl | |
| O | O | 6-$CH_3$ | 4-Methyl benzyl | Methyl | |
| O | O | H | H | 2,4,6-Trimethylphenyl | 212—213 |
| O | O | 4-Cl | H | Methyl | 122—124 |
| O | O | H | H | 3,4-Difluorphenyl | |
| O | O | 6-$NO_2$ | $C_2H_5$ | 2,5-Dichlorbenzyl | |
| O | O | H | H | 5-Indanyl (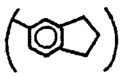) | 171—173 |
| O | O | 4-$CH_3$ | H | Äthyl | 80— 81 |
| O | O | H | H | 3-Isopropylphenyl | 98—100 |
| S | O | H | $C_2H_5OCH_2$ | n-Butyl | |
| O | O | H | H | Cyclooctyl | 103—105 |
| O | O | H | H | 4-Methylphenyl | 138—139 |
| O | O | H | H | 2,4-Dibromphenyl | |
| O | O | H | H | 3-Methyl-5-äthylphenyl | 115—117 |
| O | O | H | H | Methoxycarbonylmethyl | 123—125 |

| A | B | X | $R^1$ | $R^3$ | Fp. °C |
|---|---|---|---|---|---|
| O | O | H | H | tert.Butyl | 97— 99 |
| O | O | H | H | 4-Äthoxyphenyl | |
| O | O | H | H | 3-Äthylphenyl | 85— 86 |
| O | O | H | $CH_3$ | Cycloheptyl | |
| O | O | H | H | 2,6-Dimethylphenyl | 165—167 |
| O | O | 5-$CF_3$ | H | Isopropyl | 121—123 |
| O | O | 6-F | H | 4-Difluormethoxyphenyl | |
| O | O | H | H | 2-Methoxyphenyl | |
| O | O | H | H | Tricyclo[4.3.$1^{2.5}O^{1.6}$] | 103—105 |
| O | O | H | H | 2-Methylphenyl | 126—128 |
| O | O | H | H | 4-Jodphenyl | |
| O | O | H | $CH_3$ | Methyl | 54— 56 |
| O | O | H | H | 3-Methyl-4-chlorphenyl | 137—138 |
| O | O | H | H | 3,5-Dimethylcyclohexyl | 128—129 |
| O | O | H | H | 1-Naphthyl | 141—142 |
| O | O | H | H | Isopropyl | 86— 89 |
| O | O | 6-Br | H | Äthyl | |
| O | O | 6-F | H | Methyl | 116—118 |
| O | O | 2-$CH_3$ | Benzyl | Äthyl | |
| O | O | 5-$CF_3$ | H | Phenyl | 133—135 |
| O | O | 4-Br | $CH_3$ | Methyl | |
| O | O | H | H | 2,6-Dimethylcyclohexyl | 121—123 |
| O | O | H | $CH_3OCH_3$— | 3-Fluorphenyl | |
| O | O | 6-$OCH_3$ | H | Methyl | 131—132 |
| O | O | 2-$CH_3$ | $CH_3$ | Benzyl | |
| O | O | 5-$CF_3$ | $CH_3$ | Phenyl | |
| O | O | H | H | Cycloheptyl | 102—104 |
| O | O | H | H | Benzyl | 113—115 |
| O | O | 4-$CH_3$<br>6-$NO_2$ | H | Methyl | |
| O | O | 6-$OCH_3$ | H | Phenyl | 209—211 |
| O | O | H | H | 4-Bromphenyl | 136—137 |

| A | B | X | $R^1$ | $R^3$ | Fp. °C |
|---|---|---|---|---|---|
| S | O | H | H | Phenyl | |
| O | O | H | H | 3-Methyl-5-isopropylphenyl | |
| O | S | H | Benzyl | n-Butyl | |
| O | O | H | H | Norbornyl | 118—120 |
| O | O | H | H | 2-Naphthyl | |
| S | S | 5-$NO_2$ | $C_2H_5$ | 3-Methylphenyl | |
| O | O | H | H | Cyclopentyl | 110—112 |
| O | O | 6-Cl | H | Methyl | 136—138 |
| O | O | H | H | 3-Methylcyclohexyl | 120—122 |
| S | S | H | H | Methyl | |
| S | O | H | $CH_3$ | Phenyl | |
| O | S | H | H | Phenyl | 156—158 |
| S | O | H | H | Äthyl | |
| S | S | H | H | Phenyl | |
| O | O | H | $C_2H_5$ | Phenyl | 56— 58 |
| O | O | H | $C_2H_5$ | 3-Methylphenyl | 75— 77 |
| O | O | H | H | 3,3,5-Trimethylcyclohexyl | 79— 82 |
| O | O | H | H | 3,4-(Tetramethylen)phenyl | 164—166 |
| O | O | H | H | Cyclohexyl | 117—118 |
| O | O | H | H | 2-Methylcyclohexyl | 100—102 |
| O | O | H | H | 1,3-Dimethoxyisopropyl | 95— 96 |
| O | O | H | H | tert.-Amyl | 62— 63 |
| O | O | H | H | 2,3,6-Trimethylphenyl | 180—182 |
| O | O | H | H | 2,3,5,6-Tetramethylphenyl | 237—238 |
| O | O | H | H | 4-Tert.-Butylphenyl | 113—115 |
| O | O | H | H | 2,3,5-Trimethylphenyl | 145—147 |
| O | O | H | H | 2-Isopropyl-5-methylphenyl | 103—105 |
| O | O | H | H | 2-tert.-Butyl-4-methylphenyl | 154—156 |
| O | O | H | H | 2,6-Dimethoxyphenyl | 155—157 |
| O | O | H | H | 3-Methylphenyl | 106—108 |
| O | O | H | $C_2H_5$ | 3-Methylphenyl | 75— 77 |

| A | B | X | $R^1$ | $R^3$ | Fp. °C |
|---|---|---|---|---|---|
| O | O | H | H | 2-Methyl-6-isopropylphenyl | 122—124 |
| O | O | H | H | 3,5-Diäthylphenyl | 128—130 |
| O | O | H | H | 1-Adamantyl | 113—115 |
| O | O | H | H | 1-Methylcyclopentyl | 57— 59 |
| O | O | H | $CH_3$ | 4-Chlorphenyl | 99—103 |
| O | O | H | H | 3,4,5-Trimethoxyphenyl | 173—175 |
| O | O | H | H | 2-Methoxy-4-tert.-butylphenyl | 151—153 |
| O | O | H | H | 2,4-ditert.-Butylphenyl | 186—187 |
| O | O | H | $CH_3$ | 2,4,6-Trimethylphenyl | 75— 77 |
| O | O | H | H | 2,6-Dichlorphenyl | 156—158 |
| O | O | H | H | 2,3-Dichlorphenyl | 166—168 |
| O | O | H | H | 2,4,6-Trichlorphenyl | 171—173 |
| O | O | H | $CH_3$ | 4-Chlorphenyl | 94— 96 |
| O | O | H | H | 2-sec.-Butylphenyl | 72— 74 |
| O | O | H | H | 2-Äthylphenyl | 115—117 |
| O | O | H | H | 2,5-Dimethylphenyl | 127—128 |
| O | O | H | H | 2-Methyl-5-isopropylphenyl | 146—147 |
| O | O | H | H | 2-Isopropylphenyl | 85— 87 |
| O | O | H | H | 4-tert.-Butylphenyl | 84— 86 |
| O | O | H | H | 4-Methylcyclohexyl | 122—126 |

II. *Aminourethane*

Beispiel D

Eine Lösung von 135 Gewichtsteilen N-(3-Nitrophenyl)-carbaminsäure-4-chlorphenylester in 900 Gewichtsteilen Tetrahydrofuran (absolut) wurden mit 3 Teilen Hydrierkatalysator (Palladium auf Tierkohle, 10% versetzt und bei Raumtemperatur und 0,02 bar $H_2$-Druck bis zur Konstanz hydriert. Zur Aufarbeitung wurde die vom Katalysator befreite und mit $MgSO_4$ getrocknete Lösung soweit vom Lösungsmittel befreit, daß sich das kristalline Reaktionsprodukt gut absaugen ließ: 186—187°C. Struktur:

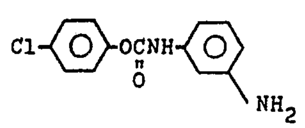

Beispiel E

Zu einer Lösung von 108 Gewichtsteilen m-Phenylendiamin in 1000 Gewichtsteilen Wasser wurden unter intensivem Rühren sehr langsam 25,2 Gewichtsteile Chlorameisensäurephenylester zugetropft. Nach beendeter Reaktion wurde abgesaugt, der Feststoff mehrmals mit verdünnter Salzsäure ausgewaschen, die vereinigten, sauren Lösungen mit Ammoniak neutralisiert und abgesaugt. Das so

erhaltene, getrocknete Produkt schmilzt bei 178 bis 180°C unter Zersetzung.
Struktur:

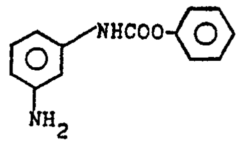

Beispiel F

Zu 51 Gewichtsteilen 2,4-Diaminonitrobenzol und 43 Gewichtsteilen Natriumhydrogencarbonat in 600 Gewichtsteilen Tetrahydrofuran tropft man langsam unter intensivem Rühren 52, 1 Gewichtsteile Chlorameisensäurephenylester. Nach 14-stündigem Rühren wird filtriert, mit Tetrahydrofuran nachgewaschen. Man befreit die Lösung soweit vom Lösungsmittel, daß sich das kristalline Rohprodukt gut absaugen läßt, nach dem Waschen mit Diäthyläther und Trocknen schmilzt die Substanz bei 223 bis 225°C. Laut NMR-Spektrum und Elementaranalyse hat sie folgende Struktur:

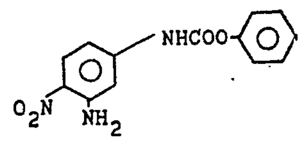

Beispiel G

Zu einer auf 80°C erwämten Mischung aus 33 Gewichtsteilen Eisenpulver, 75 Gewichtsteilen Alkohol, 60 Gewichtsteilen Wasser und 3 Gewichtsteilen konz. Salzsäure gibt man unter intensivem Rühren 40 Gewichtsteile 3-(S-Methylthiocarbamoyl)-nitrobenzol in solchen Portionen, daß die Temperatur ohne zusätzliche Heizung auf 80°C gehalten wird. Danach kocht man noch 1 Stunde am Rückfluß, saugt heiß ab, digeriert den Rückstand und das Filtrat mit etwa 1000 Gewichtsteilen Methylenchlorid, trocknet über Natriumsulfat, engt ein und kristallisiert aus Toluol um: Fp. 101—103°C.
Struktur:

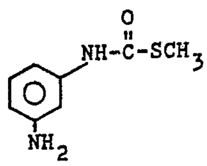

Nach entsprechenden Verfahren können folgende Aminourethane (F) hergestellt werden:

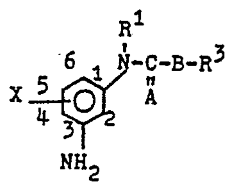

| A | B | X | $R^1$ | $R^3$ | Fp. °C |
|---|---|---|---|---|---|
| O | O | H | H | 4-Fluorphenyl | 166—167 |
| O | O | H | H | 2,4-Dichlorphenyl | 126—128 |
| O | O | H | H | Methyl | 87— 89 |
| O | O | 6-$CH_3$ | H | Methyl | |
| O | O | H | Benzyl | 4-Chlorphenyl | |
| O | O | 4-$NO_2$ | H | Methyl | 187—189 |
| O | O | H | H | 3-Methoxyphenyl | |
| O | O | H | $CH_3$ | Phenyl | 70— 72 |

| A | B | X | $R^1$ | $R^3$ | Fp. °C |
|---|---|---|---|---|---|
| O | O | H | H | 2-Fluorphenyl | 172—173 |
| C | O | 6-F | H | Phenyl | |
| O | O | H | H | 3-Bromphenyl | |
| O | O | H | $CH_3OCH_2$ | 3-Methylphenyl | |
| S | O | H | H | Methyl | |
| S | S | H | $CH_3$ | Phenyl | |
| O | S | H | H | Äthyl | |
| O | S | H | H | 3-Methylphenyl | |
| S | O | H | H | 4-Chlorphenyl | |
| S | O | H | H | Phenyl | |
| O | O | H | H | 3,4-Dimethylphenyl | 155—157 |
| O | O | 5-$CF_3$ | H | Methyl | |
| O | O | H | H | 4-Methoxyphenyl | 146—149 |
| O | O | 6-$CH_3$ | H | Äthyl | |
| O | O | H | H | 3-Fluorphenyl | Zers. |
| O | O | H | H | Äthyl | zähes Öl |
| O | O | 2-$CH_3$ | H | Phenyl | 131—133 |
| O | O | H | H | 2-Chlor-4-fluorphenyl | Zers. |
| O | O | H | H | 2-Chlorphenyl | Zers. |
| O | O | 4,6-$F_2$ | H | Methyl | |
| O | O | 4-$CH_3$ | H | Methyl | |
| S | O | 2,5-$Cl_2$ | $CH_3$ | 2-Methoxyäthyl | |
| O | O | H | H | 4-Äthylphenyl | 160—161 |
| O | O | 4-Cl | H | Phenyl | 215—217 |
| O | O | H | H | 3-Chlor-4-fluorphenyl | |
| S | S | 6-$CH_3$ | 4-Methyl-benzyl | Methyl | |
| O | O | H | H | 2,4,6-Trimethylphenyl | 150—152 |
| O | O | 4-Cl | H | Methyl | |
| O | O | H | H | 3,4-Difluorphenyl | |
| O | S | 6-$NO_2$ | $C_2H_5$ | 2,5-Dichlorbenzyl | |
| O | O | H | H | 5-Indanyl (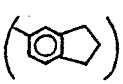) | 184—186 |

| A | B | X | $R^1$ | $R^3$ | Fp. °C |
|---|---|---|---|---|---|
| O | O | 4-$CH_3$ | H | Äthyl | |
| O | O | H | H | 3-Isopropylphenyl | 68— 70 |
| O | O | H | H | 3-Äthyl-5-methylphenyl | 102—104 |
| S | S | H | H | Phenyl | |
| O | O | H | H | 3,3,5-Trimethylcyclohexyl | 100—102 |
| O | O | H | H | 2-Methylcyclohexyl | |
| O | O | H | $C_2H_5$ | 3-Methylphenyl | 104—105 |
| O | O | H | $C_2H_5$ | Phenyl | 104—106 |
| S | O | H | $C_2H_5OCH_2$— | n-Butyl | |
| O | O | H | H | Cyclooctyl | 77— 79 |
| O | O | H | H | 4-Methylphenyl | 158—162 |
| O | O | H | H | 2,4-Dibromphenyl | |
| O | O | H | H | tert.Butyl | 109—110 |
| O | S | H | H | 4-Äthoxyphenyl | |
| O | O | H | H | 3-Äthylphenyl | 112—114 |
| O | O | H | $CH_3$ | Cycloheptyl | |
| O | O | H | H | 2,6-Dimethylphenyl | 160—161 |
| O | O | 5-$CF_3$ | H | Isopropyl | 102—104 |
| S | O | 6-F | H | 4-Difluormethoxyphenyl | |
| O | O | H | H | 2-Methoxyphenyl | |
| O | O | H | H | Tricyclo 4.3.1$^{2,5}$O$^{1,6}$-decyl | 130—131 |
| O | O | H | H | 2-Methylphenyl | 170—172 |
| O | O | H | H | 4-Jodphenyl | |
| O | O | H | $CH_3$ | Methyl | |
| O | O | H | H | 3-Methyl-4-chlorphenyl | 181 |
| O | O | H | H | 3,5-Dimethylcyclohexyl | 80— 82 |
| O | O | H | H | 1-Naphthyl | 146—148 |
| O | O | H | H | Isopropyl | 66— 68 |
| O | S | 6-Br | H | Äthyl | |
| S | O | H | H | 4-Nitrophenyl | |
| O | O | 6-F | H | Methyl | |

| A | B | X | $R^1$ | $R^3$ | Fp. °C |
|---|---|---|---|---|---|
| O | O | 2-$CH_3$ | Benzyl | Äthyl | |
| O | O | 5-$CF_3$ | H | Phenyl | 214—216 |
| S | S | 4-Br | $CH_3$ | Methyl | |
| O | O | H | H | 2,6-Dimethylcyclohexyl | |
| O | O | H | H | 2-Äthylhexyl | zähes Öl |
| O | S | H | H | Phenyl | |
| S | O | H | $CH_3OCH_2$— | 3-Fluorphenyl | |
| O | O | 6-$OCH_3$ | H | Methyl | |
| O | S | 2-$CH_3$ | $CH_3$ | Benzyl | |
| O | S | 5-$CF_3$ | $CH_3$ | Phenyl | |
| O | O | H | H | Cycloheptyl | 86— 88 |
| O | O | H | H | Benzyl | |
| S | O | 4-$CH_3$ 6-$NO_2$ | H | Methyl | |
| O | O | 6-$OCH_3$ | H | Phenyl | 84— 86 |
| O | O | H | H | 3-Methyl-5-isopropylphenyl | |
| O | S | H | Benzyl | n-Butyl | |
| O | O | H | H | Norbornyl | 133—135 |
| O | O | H | H | 2-Naphthyl | |
| O | S | 5-$NO_2$ | $C_2H_5$ | 3-Methylphenyl | |
| O | O | H | H | Cyclopentyl | |
| O | O | 6-Cl | H | Methyl | |
| O | O | H | H | 3-Methylcyclohexyl | 95— 97 |
| O | O | H | $CH_3$ | 3-Methylphenyl | 112—115 |
| O | O | H | H | 3,4-(Tetramethylen)-phenyl | 181—183 |
| S | S | H | H | Methyl | |
| O | O | H | H | 2-Isopropyl-5-methylphenyl | 122—123 |
| O | O | H | H | 3-Methylphenyl | 142—144 |
| O | O | H | H | 2-tert.-Butyl-4-methylphenyl | 89— 91 |
| O | O | H | H | tert.-Amyl | 65— 67 |
| O | O | H | H | 4-tert.-Butylphenyl | 175—177 |
| O | O | H | H | 2,3,5-Trimethylphenyl | 152—154 |

| A | B | X | $R^1$ | $R^3$ | Fp. °C |
|---|---|---|---|---|---|
| O | O | H | H | 2,3,6-Trimethylphenyl | 155—156 |
| O | O | H | H | 3,5-Diäthylphenyl | 121—123 |
| O | O | H | H | Cyclohexyl | 122—124 |
| O | O | H | H | 2-Methylcyclohexyl | |
| O | O | H | H | 1,3-Dimethoxyisopropyl | |
| O | O | H | H | 2-Methyl-6-isopropylphenyl | 133—135 |
| O | O | H | $CH_3$ | 2,4,6-Trimethylphenyl | |
| O | O | H | H | 4-Methylcyclohexyl | 73— 75 |
| O | O | H | H | 1-Adamantyl | 158—161 |
| O | O | H | H | 1-Methylcyclopentyl | |
| O | O | H | $CH_3$ | 4-Chlorphenyl | 88— 90 |
| O | O | H | H | 3,4,5-Trimethoxyphenyl | 146—148 |
| O | O | H | H | 2-Methoxy-4-methylphenyl | 110—112 |
| O | O | H | H | 2-Methyl-4-tert.-butylphenyl | 185—186 |
| O | O | H | $CH_3$ | 2,4,6-Trimethylphenyl | |
| O | O | H | H | 2,4-Di-tert.-butylphenyl | 195—197 |
| O | O | H | H | 2-sec.-Butylphenyl | 75— 77 |
| O | O | H | H | 2-Äthylphenyl | 74— 75 |
| O | O | H | $CH_3$ | 4-Fluorphenyl | 123—125 |
| O | O | H | H | 2,5-Dimethylphenyl | 142—144 |
| O | O | H | H | 2-Methyl-5-isopropylphenyl | 139—141 |
| O | O | H | H | 2,3-Dimethylphenyl | 184—186 |
| O | O | H | H | 2-Isopropylphenyl | 80— 82 |

III. *Diurethane*

Beispiel 1

Einer Lösung von 22,8 Gewichtsteilen N-(3-Aminophenyl)-carbaminsäurephenylester in 200 Gewichtsteilen Tetrahydrofuran (absolut) wurden 11 Gewichtsteile Natriumcarbonat zugegeben und anschließend bei 20 bis 25°C unter Kühlung 13,3 Gewichtsteile Chlorameisensäurethiomethylester zudosiert. Zur Vervollständigung der Umsetzung wurde 1 Stunde bei Raumtemperatur gerührt. Anschließend wurde die Reaktionsmischung filtriert und das Filtrat im Vakuum eingleinget. Der ölige Rückstand wurde durch Zugabe von Toluol zur Kristallisation gebracht.
Fp. 155—157°C (Nr. 1).

Die Verbindung hat folgende Strukturformel:

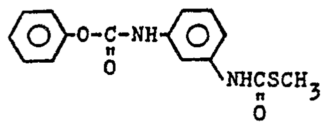

Beispiel 2

Eine Mischung aus 20 Gewichtsteilen N-(3-Isothiocyanatophenyl)-O-methylcarbamat (erhältich aus N-(3-Aminophenyl)-O-methylcarbamat und Thiophosgen, Fp. 99—100°C, 20 Gewichtsteilen Methanol, 3 Gewichtsteilen Triäthylamin und 150 Gewichtsteilen Toluol wird 6 Stunden zum Sieden erhitzt. Nach dem Einengen kristallisiert man aus Toluol um: Fp. 147—149°C (Nr. 2).

Die Verbindung hat folgende Strukturformel:

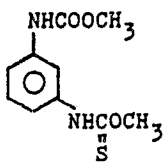

Beispiel 3

Zu einer Lösung von 16,6 Gewichtsteilen N-(3-Aminophenyl)-O-methylurethan und 10,1 Gewichtsteilen Triäthylamin in 300 Gewichtsteilen Diäthyläther läßt man bei Raumtemperatur 10 Gewichtsteile Schwefelkohlenstoff tropfen. Nach 20-stündigem Rühren wird abgesaugt, der Rückstand in 120 Gewichtsteilen Wasser suspendiert und unter Rühren mit 9,1 Volumenteilen Diäthylsulfat versetzt. Nach 20-stündigem Rühren wird abgesaugt, mit Wasser gewaschen und an der Luft getrocknet: Fp. 122—124°C (Nr. 3).

Die Verbindung hat folgende Strukturformel:

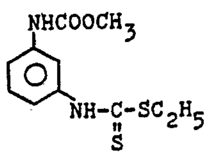

In entsprechender Weise können die folgenden Verbindungen hergestellt werden:

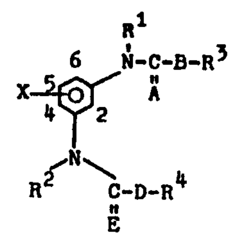

| Nr. | A | B | D | E | X | $R^1$ | $R^2$ | $R^3$ | $R^4$ | Fp. °C |
|---|---|---|---|---|---|---|---|---|---|---|
| 4 | O | S | O | O | H | H | H | Äthyl | Phenyl | 162—164 |
| 5 | O | S | O | O | 2-$CH_3$ | H | H | Methyl | Phenyl | 180—182 |
| 6 | O | O | S | O | H | H | H | Methyl | Phenyl | 155—157 |
| 7 | O | O | S | O | H | H | H | Phenyl | n-Propyl | 120—122 |
| 8 | O | O | S | O | H | H | H | Methyl | 4-Chlor-phenyl | 197—198 |
| 9 | O | S | O | O | H | H | $CH_3$ | Methyl | 3-Methylphenyl | 118—120 |
| 11 | S | S | O | O | H | H | H | Methyl | 3-Äthylphenyl | 135—138 |
| 12 | O | S | O | O | 5-$CF_3$ | H | H | Methyl | Phenyl | 68— 71 |
| 13 | O | O | S | O | H | H | H | Methyl | Methyl | 144—146 |
| 14 | O | O | S | O | H | $CH_3$ | H | 3-Methylphenyl | Äthyl | 120—121 |
| 15 | O | O | S | S | H | H | H | Methyl | Methyl | 148—150 |
| 16 | O | S | O | O | H | H | H | n-Butyl | Phenyl | 127—129 |
| 17 | S | S | O | O | H | H | $CH_3$ | Methyl | 3-Methylphenyl | zähes Öl |
| 18 | O | S | O | O | 4-$OCH_3$ | H | H | Methyl | Phenyl | 123—125 |
| 19 | O | O | O | S | H | H | H | Methyl | 4-Chlorphenyl | 156—158 |
| 20 | O | O | S | O | 6-Cl | H | H | Phenyl | Methyl | 183—185 |

| Nr. | A | B | D | E | X | $R^1$ | $R^2$ | $R^3$ | $R^4$ | Fp. °C |
|---|---|---|---|---|---|---|---|---|---|---|
| 21 | O | S | S | O | H | H | H | Methyl | Methyl | 186—188 |
| 24 | O | S | O | O | H | H | H | Phenyl | Phenyl | 158—159 |
| 25 | O | S | O | O | H | H | Benzyl | Methyl | 4-Chlorphenyl | |
| 26 | O | S | O | O | H | H | $CH_3$ | Äthyl | Phenyl | 131—133 |
| 27 | O | O | S | O | 5-$CF_3$ | H | H | Isopropyl | Phenyl | 135—137 |
| 28 | O | O | S | O | H | $CH_3OCH_2$ | H | 3-Methylphenyl | Methyl | |
| 29 | O | S | O | O | H | H | H | Methyl | 2,4-Dichlorphenyl | 167—168 |
| 30 | O | S | O | O | H | H | H | 2-Phenyläthyl | Phenyl | 129—131 |
| 31 | O | O | O | S | H | H | H | Äthyl | 3-Bromphenyl | 163—165 |
| 32 | O | S | O | O | H | H | H | Methyl | 3-Bromphenyl | 163—165 |
| 33 | O | O | S | O | 6-$OCH_3$ | H | H | Methyl | Äthyl | 115—117 |
| 34 | O | S | O | O | H | H | H | Methyl | 3-Methoxyphenyl | |
| 35 | O | S | O | O | H | H | H | Äthyl | 3-Äthylphenyl | 130—132 |
| 36 | S | S | O | O | H | H | $CH_3$ | Methyl | Phenyl | 96— 97 |
| 37 | O | O | S | O | H | H | H | 4-Methylphenyl | Äthyl | 167—169 |
| 39 | S | S | O | O | H | H | H | Methyl | 4-Methylphenyl | 142—144 |
| 40 | S | O | O | O | 3,5-$Cl_2$ | H | $CH_3$ | 1-Naphthyl | 2-Methoxyäthyl | |
| 41 | S | O | O | O | H | H | H | Phenyl | Methyl | 164—167 |
| 42 | O | O | S | O | H | $C_2H_5$ | H | 3-Methylphenyl | Äthyl | |
| 43 | O | O | S | S | H | H | H | Methyl | Phenyl | 148—150 |
| 44 | S | O | O | O | H | H | H | 4-Trifluormethyl | Methyl | |

| Nr. | A | B | D | E | X | $R^1$ | $R^2$ | $R^3$ | $R^4$ | Fp. °C |
|---|---|---|---|---|---|---|---|---|---|---|
| 45 | O | O | S | S | H | H | H | 4-Fluorphenyl | Methyl | 135—138 |
| 46 | S | S | S | S | H | H | H | Methyl | Phenyl | |
| 47 | S | S | O | O | H | H | H | Methyl | Phenyl | 134—136 |
| 49 | O | S | O | O | H | H | H | Äthyl | 4-Fluorphenyl | 152—154 |
| 51 | O | O | O | S | H | H | H | Methyl | 4-Methylphenyl | 148—150 |
| 52 | O | O | S | O | H | H | H | 3-Chlor-4-fluor-phenyl | Methyl | |
| 53 | O | S | O | O | H | H | H | Methyl | 4-tert.-Butyl-cyclohexyl | 117—119 |
| 54 | O | O | S | O | H | H | H | tert.Butyl | Äthyl | 128—130 |
| 55 | O | S | O | O | H | H | 4-Methylbenzyl | Methyl | Phenyl | |
| 56 | O | S | O | O | H | H | H | Äthyl | 4-tert.-Butyl-cyclohexyl | 103—105 |
| 58 | O | O | S | O | H | H | H | Norbornyl | Methyl | 139—141 |
| 59 | O | O | S | O | H | H | H | Cyclooctyl | Methyl | 138—140 |
| 60 | O | S | O | O | H | $CH_3$ | H | Methyl | Phenyl | |
| 61 | O | S | O | O | H | H | $CH_3$ | Methyl | Phenyl | 118—120 |
| 62 | O | O | S | O | H | H | H | 3,4-Difluorphenyl | Methyl | |
| 63 | O | S | S | O | H | H | H | Phenyl | Methyl | 148—150 |
| 64 | O | O | S | O | H | H | H | Methyl | 2,4-Dichlorbenzyl | |
| 65 | O | O | S | O | H | H | H | Methyl | Benzyl | 117—119 |
| 67 | O | S | O | O | H | H | $C_2H_5$ | Methyl | 3-Methylphenyl | 128—130 |
| 68 | O | O | S | O | H | H | H | Phenyl | Benzyl | 148—149 |

| Nr. | A | B | D | E | X | $R^1$ | $R^2$ | $R^3$ | $R^4$ | Fp. °C |
|---|---|---|---|---|---|---|---|---|---|---|
| 69 | O | S | O | O | H | H | H | Methyl | Cyclopentyl | |
| 70 | O | S | O | O | H | H | H | Methyl | tert.-Butyl | 142—144 |
| 71 | S | S | S | O | 4-$CH_3$ | H | H | Methyl | Phenyl | |
| 72 | O | S | O | S | 6-$CH_3$ | H | H | Phenyl | Phenyl | |
| 73 | O | S | O | O | H | H | H | Methyl | 1-Naphthyl | 195—196 |
| 74 | S | O | S | S | H | H | H | Propargyl | Methyl | |
| 75 | O | S | O | O | 2-$CH_3$ | H | H | Äthyl | 4-tert.-Butylphenyl | |
| 76 | O | S | O | O | H | H | H | Methyl | 2-Fluorphenyl. | 183—185 |
| 77 | O | S | S | S | H | H | H | n-Propyl | Phenyl | |
| 78 | O | O | S | O | H | H | H | 3-Fluorphenyl | Methyl | 161—162 |
| 79 | S | O | O | S | H | H | H | Methyl | Phenyl | |
| 80 | O | S | O | O | H | H | H | Methyl | 4-Fluorphenyl | 171—173 |
| 81 | S | S | O | S | H | H | H | Methyl | Phenyl | |
| 82 | O | O | S | O | H | H | H | 2-Chlorphenyl | Methyl | 183—184 |
| 83 | S | S | S | O | H | H | H | Methyl | Phenyl | |
| 84 | O | S | O | O | 4-$CH_3$ | H | H | Methyl | 4-Methylphenyl | |
| 85 | O | O | S | O | H | H | H | 2-Methylphenyl | Methyl | 176—177 |
| 86 | S | O | O | S | H | H | H | Methyl | 2-Methylphenyl | |
| 87 | O | S | O | O | H | H | H | Methyl | 4-Methylphenyl | 188—190 |
| 88 | O | S | O | S | H | H | H | Phenyl | Methyl | |
| 89 | O | S | S | S | H | H | H | 4-Chlorphenyl | Methyl | |

| Nr. | A | B | D | E | X | $R^1$ | $R^2$ | $R^3$ | $R^4$ | Fp. °C |
|---|---|---|---|---|---|---|---|---|---|---|
| 90 | O | S | O | O | H | H | H | Methyl | 3-Äthylphenyl | 118—120 |
| 91 | O | S | S | S | 6-$CH_3$ | H | H | 4-Chlorphenyl | Äthyl | |
| 92 | O | S | O | O | H | H | H | Methyl | 4-Äthylphenyl | 186—188 |
| 93 | O | S | O | S | H | H | H | Methyl | 4-Chlorphenyl | |
| 95 | S | O | O | O | 4-$NH_2$ | H | H | sec. Butyl | Propargyl | |
| 96 | O | S | O | O | H | H | H | Methyl | 4-Methoxyphenyl | 185—186 |
| 97 | S | O | O | S | H | H | H | sec.-Butyl | Methyl | |
| 98 | O | S | O | O | H | H | H | Methyl | 3,4-Dimethyl-phenyl | 180—182 |
| 99 | S | O | S | O | H | H | H | 3-Methylphenyl | Methyl | |
| 100 | O | O | S | O | H | H | H | 2-Chlor-4-fluor-phenyl | Methyl | 183—184 |
| 101 | S | S | S | O | 4-$OCH_3$ | H | H | Methyl | Phenyl | |
| 102 | O | S | O | O | H | H | H | Äthyl | 3-Isopropylphenyl | 107—109 |
| 103 | O | O | S | O | H | H | H | 2,6-Dimethyl-phenyl | Methyl | 110—112 |
| 105 | O | S | O | O | H | H | H | Methyl | 2,4,6-Trimethyl-phenyl | 188—190 |
| 106 | O | O | S | O | H | $CH_3$ | H | 4-Methylphenyl | Äthyl | |
| 107 | O | S | O | O | H | H | H | Methyl | 5-Indanyl | 193—194 |
| 108 | S | S | O | S | 4-$NO_2$ | H | H | Methyl | Methyl | |
| 110 | S | O | O | O | H | H | H | Phenyl | Methoxycarbonyl-methyl | |

| Nr. | A | B | D | E | X | $R^1$ | $R^2$ | $R^3$ | $R^4$ | Fp. °C |
|---|---|---|---|---|---|---|---|---|---|---|
| 111 | O | S | O | O | H | H | H | Methyl | 3,4-(Tetra-methylen)-phenyl | 163—165 |
| 113 | O | O | S | S | H | H | H | 3,4-(Tetra-methylen)-phenyl | Methyl | |
| 114 | O | S | O | S | H | H | H | Methyl | 2-Naphthyl | |
| 115 | O | S | O | O | H | H | H | Methyl | 3-Methyl-5-äthylphenyl | 120—122 |
| 116 | O | O | S | O | H | H | H | 3,4-(Tetra-methylen)-phenyl | Äthyl | |
| 117 | S | O | S | S | H | H | H | Methyl | Phenyl | |
| 118 | O | S | O | O | H | H | H | Methyl | 3-Isopropyl-phenyl | 104—106 |
| 119 | S | S | O | S | 6-$NO_2$ | H | H | Methyl | 4-Methylphenyl | |
| 120 | O | O | S | O | H | H | H | 3-Methyl-5-äthylphenyl | Äthyl | |
| 121 | O | O | O | S | H | H | H | 2-Naphthyl | Methyl | |
| 124 | | | | | | | | | | |
| 125 | S | S | S | O | H | H | H | Methyl | 4-Methylphenyl | |
| 126 | S | S | O | O | H | H | H | Methyl | tert.-Butyl | 101—103 |
| 127 | O | S | O | S | 6-Br | H | H | Allyl | tert.-Butyl | |
| 128 | O | S | S | O | H | $CH_3$ | H | Phenyl | Äthyl | |
| 129 | O | O | S | O | H | H | H | 3,3,5-Trimethyl-cyclohexyl | Äthyl | Öl |
| 130 | S | O | O | O | H | H | H | Phenyl | tert.-Butyl | 143—147 |

| Nr. | A | B | D | E | X | $R^1$ | $R^2$ | $R^3$ | $R^4$ | Fp. °C |
|---|---|---|---|---|---|---|---|---|---|---|
| 131 | O | O | S | O | H | Isopropyl | H | Phenyl | Methyl | |
| 132 | O | S | O | O | H | H | H | Methyl | Cyclohexyl | 145—146 |
| 133 | O | S | S | O | H | $CH_3$ | H | Methyl | Phenyl | |
| 134 | O | S | O | O | H | H | H | Methyl | 3,5-Dimethyl-cyclohexyl | 129—131 |
| 135 | O | O | S | S | H | H | H | tert.-Butyl | Phenyl | |
| 136 | S | S | O | S | 4-F | H | H | Methyl | Benzyl | |
| 137 | O | S | O | O | H | H | H | Äthyl | Tricyclo-(4.3.$1^{2,5}O^{1,6}$)-decyl | Öl |
| 138 | O | O | S | O | H | H | H | 3-Methyl-cyclohexyl | Äthyl | Öl |
| 139 | S | S | S | O | H | H | H | Methyl | n-Butyl | |
| 140 | O | S | O | O | H | H | Benzyl | Methyl | 4-Fluorphenyl | |
| 141 | O | S | S | S | H | H | H | Methyl | Phenyl | 155—157 |
| 142 | S | O | S | S | 4,6-$F_2$ | H | H | 4-Jodphenyl | Methyl | |
| 143 | O | O | S | O | H | H | H | tert.Butyl | Phenyl | |
| 144 | O | S | O | O | H | H | H | Methyl | 3-Methyl-4-chlorphenyl | 162—164 |
| 145 | O | S | O | O | H | H | H | Äthyl | 2,6-Dimethyl-cyclohexyl | |
| 146 | O | S | S | O | H | $CH_3$ | H | Phenyl | Methyl | |
| 148 | O | O | S | O | H | H | H | 2-Äthylhexyl | Äthyl | Öl |
| 149 | O | S | O | O | H | H | H | Methyl | 2,6-Dimethyl-cyclohexyl | 64— 66 |

| Nr. | A | B | D | E | X | $R^1$ | $R^2$ | $R^3$ | $R^4$ | Fp. °C |
|---|---|---|---|---|---|---|---|---|---|---|
| 150 | O | S | O | O | H | H | H | Methyl | 3,5,5-Trimethyl-cyclohexyl | 57— 60 |
| 151 | O | S | O | O | H | H | H | Methyl | 3-Methylcyclo-hexyl | 98—100 |
| 152 | O | S | O | O | H | H | H | Methyl | Cycloheptyl | |
| 153 | O | S | O | O | H | H | H | Äthyl | Cycloheptyl | |
| 155 | O | S | O | O | H | $CH_3$ | H | Methyl | Methyl | |
| 158 | O | O | S | O | H | $CH_3$ | H | Methyl | Phenyl | 163—165 |
| 160 | O | S | S | S | H | H | H | Isopropyl | Phenyl | |
| 161 | S | S | S | O | H | H | H | Methyl | Methyl | 146—148 |
| 163 | S | O | S | O | H | H | H | Phenyl | Methyl | 186—188 |
| 166 | O | S | O | O | H | H | $C_2H_5$ | Äthyl | Phenyl | 140—142 |
| 167 | S | O | O | S | H | H | H | Methyl | Cyclohexyl | |
| 169 | O | O | S | O | H | $ClCH_2$— | H | Phenyl | Methyl | |
| 170 | S | O | S | O | H | H | H | 4-Methylphenyl | Methyl | 191—193 |
| 172 | O | S | O | O | H | H | H | Methyl | Propargyl | 104—105 |
| 174 | O | S | O | O | H | H | H | Methyl | 2-Äthylhexyl | 89— 91 |
| 175 | S | O | S | S | H | H | H | sec.-Butyl | n-Butyl | |
| 176 | O | O | S | O | H | $C_2H_5$ | H | Phenyl | Methyl | 140—142 |
| 178 | O | S | O | O | H | H | H | Phenyl | Äthyl | |
| 179 | S | O | O | O | H | H | H | Phenyl | Äthyl | |
| 180 | S | O | O | O | H | H | H | Phenyl | Isopropyl | |

| Nr. | A | B | D | E | X | $R^1$ | $R^2$ | $R^3$ | $R^4$ | Fp. °C |
|---|---|---|---|---|---|---|---|---|---|---|
| 181 | O | S | O | O | H | H | H | Methyl | Isopropyl | 135—136 |
| 182 | S | S | S | S | H | H | H | 3-Methylphenyl | Allyl | |
| 183 | O | S | O | O | H | H | $CH_3OCH_2$ | Methyl | Phenyl | |
| 184 | O | S | O | O | H | $CH_3$ | H | Phenyl | Methyl | |
| 186 | S | O | O | O | H | H | H | 4-Methylphenyl | Äthyl | |
| 187 | O | S | O | O | H | H | $CH_3$ | Methyl | 4-Chlorphenyl | 149—152 |
| 188 | O | O | S | O | H | $CH_3$ | H | 4-Methylphenyl | Methyl | |
| 189 | O | O | S | O | H | H | $CH_3$ | Methyl | 4-Chlorphenyl | |
| 190 | O | S | S | O | H | H | $CH_3$ | Phenyl | Äthyl | |
| 191 | O | O | S | O | H | H | $CH_3$ | Phenyl | Äthyl | |
| 192 | O | S | o | O | H | H | $CH_3$ | Phenyl | Äthyl | |
| 194 | S | S | S | S | H | H | H | Methyl | 4-Fluorphenyl | |
| 195 | O | O | S | O | H | H | $CH_3$ | Methyl | 4-Methylphenyl | |
| 196 | O | O | O | S | H | H | H | Phenyl | Methyl | 155 |
| 197 | S | O | O | O | 6-Cl | H | H | Phenyl | Methyl | 135—137 |
| 198 | O | S | O | O | H | H | H | Methyl | Äthyl | |
| 199 | O | S | O | O | H | H | H | Methyl | 2,3,6-Trimethylphenyl | 196—198 |
| 200 | O | S | O | O | H | H | $CH_3$ | Methyl | Methyl | |
| 201 | O | O | S | O | H | H | H | 2,3,5-Trimethylphenyl | Methyl | 189—191 |
| 202 | O | S | O | O | H | H | $CH_3$ | Methyl | 2,3,5-Trimethylphenyl | |

| Nr. | A | B | D | E | X | $R^1$ | $R^2$ | $R^3$ | $R^4$ | Fp. °C |
|---|---|---|---|---|---|---|---|---|---|---|
| 203 | O | S | O | O | H | H | $CH_3$ | Methyl | 2,3,6-Trimethyl-phenyl | |
| 204 | O | S | O | O | H | H | $CH_3$ | Methyl | 2,4,6-Trimethyl-phenyl | 196—198 |
| 205 | S | S | O | O | H | H | $CH_3$ | Methyl | 2,4,6-Trimethyl-phenyl | 157—159 |
| 206 | O | O | S | O | H | H | H | tert.-Amyl | Methyl | 91— 93 |
| 207 | O | S | O | O | H | H | H | Methyl | 1,3-Dimethoxy-isopropyl | |
| 208 | O | O | S | O | H | H | H | 1-Chlorisopropyl | Methyl | |
| 210 | O | O | S | O | H | H | H | Benzyl | Methyl | 146—147 |
| 211 | O | S | O | O | H | H | H | Methyl | 1,1-Dimethyl-2-chloräthyl | 117—120 |
| 212 | O | O | S | O | H | H | H | 4-Methylcyclo-hexyl | Methyl | 152—155 |
| 213 | O | S | O | O | H | H | H | Methyl | 2-Methylcyclo-hexyl | |
| 214 | O | O | S | O | H | H | H | 1-Methyl-cyclopentyl | Methyl | 89— 92 |
| 215 | O | O | S | O | H | H | H | 1-Adamantyl | Methyl | 134—137 |
| 216 | O | S | O | O | H | H | H | Methyl | Butin-1-yl-3 | 128—129 |
| 217 | O | S | S | O | H | H | H | Benzyl | Methyl | 125—127 |
| 218 | O | S | O | O | H | H | H | Methyl | 3-Methylphenyl | 145—147 |
| 219 | O | S | O | O | H | H | H | Äthyl | 3-Methylphenyl | 126—128 |
| 220 | O | O | S | O | H | H | H | 2-Äthylphenyl | Methyl | 160—162 |

| Nr. | A | B | D | E | X | $R^1$ | $R^2$ | $R^3$ | $R^4$ | Fp. °C |
|---|---|---|---|---|---|---|---|---|---|---|
| 221 | O | S | O | O | H | H | H | Methyl | 2-Isopropylphenyl | 125—127 |
| 222 | O | S | O | O | H | H | H | Methyl | 4-Isopropylphenyl | 145—147 |
| 223 | O | S | O | O | H | H | H | Methyl | 4-tert.-Butylphenyl | 100—102 |
| 224 | O | S | O | O | H | H | H | Äthyl | 4-tert.-Butylphenyl | 140—142 |
| 225 | O | S | O | O | H | H | H | Methyl | 2-sec.-Butylphenyl | 127—129 |
| 226 | O | S | O | O | H | H | H | Methyl | 4-Bromphenyl | 186—188 |
| 228 | O | O | S | O | H | H | H | 3,4,5-Trimethoxy-phenyl | Methyl | 155—157 |
| 229 | O | O | S | O | H | H | H | 2-Isopropyl-5-methylphenyl | Methyl | 162—164 |
| 230 | O | S | O | O | H | H | H | Methyl | 2-tert.-Butyl-4-methylphenyl | 171—173 |
| 231 | O | S | O | O | H | H | H | Methyl | 2-Methyl-6-iso-propylphenyl | 152—153 |
| 232 | O | O | S | O | H | H | H | 3,5-Diäthylphenyl | Methyl | 132—134 |
| 233 | O | O | S | O | H | H | H | 2-Methyl-4-tert.-butylphenyl | Methyl | 150—152 |
| 234 | O | S | O | O | H | H | H | Methyl | 2,4-Ditert.-Butylphenyl | 157—158 |
| 235 | O | S | O | O | H | H | H | Methyl | 2-Methyl-5-iso-propylphenyl | 127—129 |
| 236 | O | O | S | O | H | H | H | 2,3-Dimethyl-phenyl | Methyl | 185—187 |
| 237 | O | O | S | O | H | H | H | 2,5-Dimethyl-phenyl | Methyl | 159—161 |

| Nr. | A | B | D | E | X | $R^1$ | $R^2$ | $R^3$ | $R^4$ | Fp. °C |
|---|---|---|---|---|---|---|---|---|---|---|
| 238 | O | S | O | O | H | H | H | Methyl | 2,3,5,6-Tetra-methylphenyl | 240—241 |
| 239 | O | S | O | O | H | H | H | Methyl | 2-Chlor-4,5-di-methylphenyl | 192—194 |
| 240 | O | O | S | O | H | H | H | 2-Methoxyphenyl | Methyl | 162—164 |
| 241 | O | S | O | O | H | H | H | Methyl | 2-Methoxy-4-methylphenyl | 140—142 |
| 243 | O | O | S | O | H | H | H | 2,4,5-Trichlor-phenyl | Methyl | 171—173 |
| 244 | O | S | O | O | H | H | H | Methyl | 2,4,5-Trichlor-phenyl | 173—175 |
| 245 | O | S | O | O | H | H | H | Methyl | 2,3-Dichlorphenyl | 178—179 |
| 246 | S | S | O | O | H | H | $CH_3$ | Methyl | 4-Chlorphenyl | 88— 90 |
| 247 | S | S | O | O | H | H | $CH_3$ | Methyl | 4-Fluorphenyl | 105—107 |
| 248 | O | S | O | O | H | H | $CH_3$ | Methyl | 4-Fluorphenyl | 153—155 |
| 249 | O | S | S | O | H | $CH_3$ | H | Methyl | Methyl | 169—171 |
| 250 | S | S | O | O | H | H | $C_2H_5$ | Methyl | Phenyl | 104—106 |
| 251 | O | S | O | O | H | H | H | Methyl | 4-sec.-Butylphenyl | |

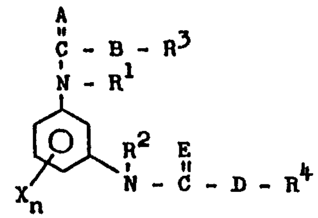

| Nr. | A | B | D | E | X | $R^1$ | $R^2$ | $R^3$ | $R^4$ | Fp. °C |
|---|---|---|---|---|---|---|---|---|---|---|
| 252 | O | O | S | S | H | H | H | 3,3-Dimethyl-5-methylcyclohexyl | $CH_3$ | 145—147 |
| 253 | O | S | O | O | H | H | $—CH_2—COOCH_3$ | $CH_3$ | Phenyl | 115—117 |
| 254 | O | O | S | O | H | H | H | $CH_3$ | tert.-Butyl | 133—135 |
| 255 | O | S | O | O | H | H | $—CH_2—CH_2Cl$ | $CH_3$ | Phenyl | |
| 256 | O | S | O | O | H | H | H | tert.Butyl | $C_2H_5$ | 96— 98 |
| 257 | O | S | O | O | H | H | H | tert.Butyl | Phenyl | 159—161 |
| 258 | O | S | S | O | H | H | H | tert.Butyl | Methyl | 132—134 |
| 259 | O | S | O | O | H | H | $CH_3$ | tert.Butyl | Phenyl | 148—150 |
| 260 | O | S | O | O | H | H | H | tert.Butyl | Norbornyl | 205—207 |
| 261 | O | S | O | O | H | H | Propyl | Methyl | Phenyl | 138—140 |

Für die folgenden Versuche wurden bekannte herbizide Wirkstoffe zum Vergleich herangezogen. 3-Methoxycarbonylaminophenyl-N-(3'Methyl-phenyl)-carbamat und 3-Äthylcarbonylaminophenyl-N-phenylcarbamat (DT-AS 15 67 151) zeichen sich durch ihre, wenngleich unterschiedliche, Wirkung gegen breitblättrige unerwünschte Pflanzen bei einem beachtlichen Maß an Verträglichkeit für Zuckerrüben aus. Hier sind bekanntlich jedoch Unterschiede zu verzeichnen, da 3-Methoxycarbonylaminophenyl-N-(3'Methyl-phenyl)-carbamat eine noch günstigere Selektivität in dieser Kultur aufweist als die zweite oben genannte Verbindung dieses Typs. Einen völlig anderen Anwendungsbereich hat 3-Isopropyl-2,1,3-benzothiadiazinon(4)-2,2-dioxid (DT-AS 15 42 836). Mit dieser Verbindung werden verschiedene breitblättrige Unkräuter in Sojabohnen, Erdnüssen, Getreide, Mais und einigen Gemüsearten bekämpft. Hier sind jedoch noch Wirkungslücken vorhanden. 3-Methoxycarbonylaminophenyl-N-(3'methyl-phenyl)-carbamat zeigt bei für seine Klasse guter herbizider Wirkung kaum Kulturpflanzenselektivität, weshalb die Verbindung z.B. für Soja nur zur Unterblattspritzung (post-directed) empfohlen wurde (Arndt, F and G. Boroschewsky; New Selective Herbicides — VIII International Plant Protection Congress, Reports and Information, Section III Chemical Control, Part I, Moskow 1975, pp. 42—49). Hierbei sollen die jungen Sproßteile und Blätter der Kulturpflanzen von der Spritzbrühe nicht getroffen werden, wohl aber unerwünschte Pflanzen, die sich unter diesen befinden.

*Beispiele zur herbiziden Wirkung neuer schwefelhaltiger Diurethane*

Zahlreiche Testergebnisse beweisen die guten herbiziden Eigenschaften der neuen Verbindungen. Sie werden hinsichtlich ihrer herbiziden Potenz und ihrer Selektivität in Kulturpflanzen anhand der biologischen Wirkung durch die folgenden Versuche charakterisiert:

*Gewächshausversuche*

Plastikblumentöpfe von 300 $cm^3$ Inhalt wurden mit lehmigem Sand gefüllt und mit den Testpflanzen nach Arten getrennt bestückt. Hierbei handelt es sich vorwiegend um die Einsaat von Samen oder auch um ein Verpflanzen bei vegetativ vermehrten Arten. Die Wirkstoffe wurden in Wasser als Verteilungsmittel suspendiert oder emulgiert und mittels feinverteilender Düsen bei Nachauflaufanwendung auf die Blätter der Testpflanzen und die noch bloß liegende Erdoberfläche gespritzt. Für die Blattbehandlung zog man die Pflanzen je nach Wuchsform in den Versuchsgefäßen erst bis zu einer Höhe von 3 bis 10 cm an und behandelte sie danach. Den Temperaturansprüchen der Testpflanzen kam man insofern entgegen, daß man sie in kühleren oder wärmeren Sektionen der Gewächshausanlagen aufstellte. Die Versuchsperiode dauerte 2 bis 4 Wochen. Während dieser Zeit wurden die Pflanzen gepflegt und ihre Reaktion auf die einzelnen Behandlungen ausgewertet. Die Aufwandmengen der Prüfsubstanzen gelten als kg/ha Aktivsubstanz. Für die Auswertung diente eine Bonitierungsskala von 0 bis 100. Dabei bedeutete 0 = keine Schädigung, 100 = Absterben der Pflanzen.

*Ergebnis*

Das Zahlenmaterial in den beigefügten Tabellen veranschaulicht die Wirkung der Wirkstoffe bei Blattbehandlung nach dem Auflaufen von Kulturpflanzen und unerwünschten Pflanzen (Tab. 2 bis 12). Bemerkenswert ist hierbei, daß die neuen Verbindungen bezüglich der herbiziden Aktivität und des Wirkungsspektrums zu den als Vergleichsmittel herangezogenen Diurethanen hin tendieren. Die Schwerpunkte in der Selektivität für Kulturpflanzen sind jedoch andere. Das läßt sich am Beispiel Sojabohnen und Getreide vortrefflich demonstrieren. Bei diesen Kulturen werden Verträglichkeitsgrade erreicht, welche dem dafür bekannten 3-Isopropyl-2,1,3-benzothiadiazinon(4)-2,2-dioxid ebenbürtig sind (Tab. 5, 6.). Daneben existiert eine Reihe von Kulturpflanzen, deren Toleranz gerade gegenüber den erfindungsgemäßen Verbindungen hervorsticht, während die geprüften Vergleichsmittel ungeeignet sind. (Tab. 2, 4, 5).

Als Applikationsmethode können zwar das Einbringen in den Boden oder die Behandlung der Bodenoberfläche in Betracht gezogen werden, aber die Behandlung aufgelaufener Pflanzen verdient den Vorzug. Auch Spezialanwendungen wie die Unterblattspritzung (post directed, lay-ba) kommen in Frage. Hierbei wird der Spritzstrahl so gelenkt, daß die Blätter aufgelaufener, empfindlicher Kulturpflanzen nach Möglichkeit nicht getroffen werden, während die Mittel auf die darunterliegende Bodenfläche oder dort wachsende unerwünschte Pflanzen gelangen.

In Anbetracht der Vielseitigkeit der Applikationsmethoden können die erfindungsgemäßen Mittel oder dieser enthaltende Mischungen außer bei den in den Tabellen aufgeführten Nutzpflanzen noch in einer weiteren großen Zahl von Kulturen zur Beseitigung unerwünschten Pflanzenwuchses eingesetzt werden.

Die Anwendungskonzentrationen können dabei von 0,1 bis 15 kg/ha und mehr betragen je nach dem Bekämpfungsobjekt.

Zur weiteren Verbreiterung des Wirkungsspektrums der neuen Einzelsubstanzen, zur Erzielung synergistischer Effekte oder zum Verbessern der Residualwirkung über den Boden lassen sich die neuen Verbindungen untereinander mischen oder zahlreiche andere herbizide oder wachstumsregulierende Verbindungen als Mischungs- und Kombinationspartner heranziehen.

Außerdem ist es möglich, die neuen erfindungsgemäßen Verbindungen allein oder in Kombination mit anderen Herbiziden auch noch mit weiteren Pflanzenschutzmitteln gemischt gemeinsam auszubringen. Hierbei ist an Mittel zur Bekämpfung von Schädlichen oder phytopathogenen Pilzen bzw. Bakterien zu denken. Von Interesse ist ferner die Mischbarkeit mit Mineralölsalzen, welche zur Behebung von Ernährungs- oder Spurenelementmängeln eingesetzt werden.

Zur Aktivierung der herbiziden Wirkung können Netz- und Haftmittel sowie nichtphytotoxische Öle zugesetzt werden.

Die Anwendung erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten, durch Versprühen, Vernebeln, Verstäuben, Verstreuen oder Gießen. Die Anwendungsformen richten sich nach den Verwendungszwecken; sie sollen in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, femer Kohlenteeröle, sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate, z.B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser usw. in Betracht.

Wäßrige Anwendungsformen können aus Emulsionskonzentrationen, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier-oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Naphthalinsulfonsäure, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenol, Alkylphenolpolyglykoläther, Tributylphenylpolyglykoläther, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver-, Streu- und Stäubemittel können durch Mischen oder gemeinsames Vermahlen der wirksamen Substanzen mit einem festen Tragerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Kalcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent.

Beispiel 4

Man vermischt 90 Gewichtsteile der Verbindung 2 mit 10 Gewichtsteilen N-Methyl-$\alpha$-pyrrolidon und erhält eine Lösung, die zur Anwendung in Form kleinster Tropfen geeignet ist.

Beispiel 5

20 Gewichtsteile der Verbindung 8 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Athylenoxid an 1 Mol Ricinusöl besteht.Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel 6

20 Gewichtsteile der Verbindung 13 werden in einer Mischung gelöst, die aus 40 Gewichtsteilen Cyclohexanon, 30 Gewichtsteilen Isobutanol, 20 Gewichtsteilen des Anlagerungsproduktes von

7 Mol Äthylenoxid an 1 Mol Isooctylphenol und 10 Gewichtsteilen des Anlagerungsproduktes von 40 Mol Äthylenoxid an 1 Mol Ricinüsol besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 7

20 Gewichtsteile der Verbindung 23 werden in einer Mischung gelöst, die aus 25 Gewichtsteilen Cyclohexanol, 65 Gewichtsteilen einer Mineralölfraktion von Siedepunkt 210 bis 280°C und 10 Gewichtsteilen des Anlagerungsproduktes von 40 Mol Athylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 8

20 Gewichtsteile des Wirkstoffs 7 mit 3 Gewichtsteilen des Natriumsalzes der Diisobutylnaphthalin- -sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 9

3 Gewichtsteile der Verbindung 46 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 10

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 11

40 Gewichtsteile des Wirkstofs 7 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 12

20 Teile des Wirkstoffs 2 werden mit 2 Teilen Calciumsalz der Dodecylbenzolsulfonsäure, 8 Teilen Fettalkahol-polyglykoläther 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Tabelle 1 — Liste der Testpflanzen

| Botanischer Name | Abkürzung in Tabellen | Deutsche Bezeichnung | Englische Bezeichnung |
|---|---|---|---|
| Amaranthus retroflexus | Amar. ret. | zurückgekrümmter Fuchsschwanz | redroot pigweed |
| Apium graveolens | Apium grav. | Sellerie | Celery |
| Arachis hypogaea | Arach. hyp. | Erdnuß | peanuts (groundnuts) |
| Carthamus tinctorius | Carth. tinct. | Färberdistel | safflower |
| Centaurea cyanus | Cent. cyan. | Kornblume | cornflower |
| Daucus carota | Daucus carot. | Möhre | carrots |
| Datura stramonium | Datura stram. | gemeiner Stechapfel | Jimsonweed |
| Desmodium tortuosum | Desmod. tort. | | Florida beggarweed |

Tabelle 1 (Fortsetzung) — Liste der Testpflanzen

| Botanischer Name | Abkürzung in Tabellen | Deutsche Bezeichnung | Englische Bezeichnung |
|---|---|---|---|
| Euphorbia helioscopia | Euphorb. heliosc. | Sonnenwolfsmilch | wart weed |
| Euphorbia geniculata | Euphorb. genic. | südamerikanische Wolfsmilchart | Southamerican member of the spurge family |
| Glycine max | Glyc. max | Soja | soybeans |
| Lamium spp. | Lamium spp. | Taubnesselarten | dead-nettle |
| Matricaria spp. | Matric. spp. | Kamillearten | chamomile |
| Mercurialis annua | Mercur. annua | einjähriges Bingelkraut | annual mercury |
| Sesbania exaltata | Sesb. exalt. | Turibaum | hemp sesbania (coffeeweed) |
| Setaria spp. | Setaria spp. | Borstenhirsearten | foxtail spp. |
| Sinapis alba | Sinap. alba | weißer Senf | white mustard |
| Stellaria media | Stell. media | Vogelsternmiere | chickweed |
| Solanum nigrum | Solan nigr. | schwarzer Nachtschatten | black nightshade |
| Triticum aestivum | Tritic. aest. | Weizen | wheat |
| Xanthium pensylvanicum | Xanth. pens. | Spitzklette | common cocklebur |
| Zea mays | Zea mays | Mais | Indian corn |
| Beta vulgaris | Beta vulg. | Zuckerrübe | sugar beets |
| Chrysanthemum segetum | Chrys. seg. | Saatwucherblume | corn marigold |
| Chenopodium album | Chen. alb. | Weißer Gänsefuß | lambsquarters (goosefoot) |
| Echinochloa crus galli | Echin. c. g. | Hühnerhirse | barnyardgrass |
| Gossypium hirsutum | Gossyp. hirs. | Baumwolle | cotton |
| Ipomoea spp. | Ipom. spp. | Prunkwindearten | morningglory |
| Oryza sativa | Oryza sat. | Reis | rice |
| Polygonum persicaria | Polyg. pers. | Flohknöterich | ladysthumb |

Tabelle 2—
Selektive Wirkungen bei Gemüsekulturen bei Nachauflaufanwendung im Gewächshaus

| Wirkstoff Nr. | kg/ha | Apium grav. | Daucus carota | Euphorbia heliosc. | Datura stram. | Lamium spp. |
|---|---|---|---|---|---|---|
| | | Testpflanzen und % Schädigung | | | | |
| 1 | 1,0 | 0 | 0 | 100 | 100 | 100+) |
| | 2,0 | 0 | 10 | 100 | 100 | 100 |
| | 4,0 | 0 | 20 | — | — | — |
| 4 | 1,0 | 0 | 0 | 75 | 100 | 13 |
| | 2,0 | 0 | 0 | 80 | 90 | 53 |
| | 4,0 | 10 | 0 | — | — | 100 |
| 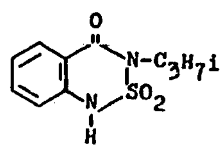 | 1,0 | 60 | 100 | 0 | 100 | 30 |
| | 2,0 | 90 | 100 | 10 | 100 | 85 |
| bekannt | 4,0 | 90 | 100 | — | — | — |
| 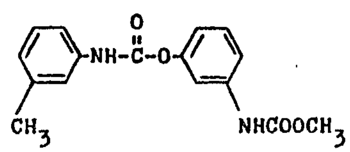 | 1,0 | 0 | 100 | 100 | 100 | 100 |
| | 2,0 | 10 | 100 | 100 | 100 | 100 |
| bekannt | 4,0 | 20 | 100 | — | — | — |

0=keine Schädigung 100=totale Schädigung +) Setaria spp. 92

Tabelle 3

Selektive herbizide Wirkung neuer Verbindungen in Getreide und Mais bei Hachauflaufanwendung im Gewächshaus

| Wirkstoff Nr. | kg/ha | Tritic. aest. | Zea mays | Cent. cyan. | Datura stram. | Lamium purp. | Matric. spp. | Mercur. annua | Sinap. alba | Stell. media |
|---|---|---|---|---|---|---|---|---|---|---|
| | | Testpflanzen und % Schädigung | | | | | | | | |
| 90 | 0,5 | 0 | 5 | — | 100 | 100 | 100 | 75 | 92 | 98 |
| | 1,0 | 0 | 15 | — | 100 | 100 | 100 | 75 | 92 | 98 |
| | 2,0 | 0 | 25 | 90 | 100 | 100 | 100 | 80 | 95 | 98 |
| 98 | 0,5 | 0 | 10 | — | 100 | 100 | 50 | 50 | 90 | 95 |
| | 1,0 | 0 | 20 | — | — | 100 | 50 | 50 | 90 | 95 |
| | 2,0 | 0 | 30 | 100 | — | 100 | 50 | 95 | 95 | 95 |
| 96 | 2,0 | 0 | — | 50 | — | 100 | — | 100 | 90 | — |
| 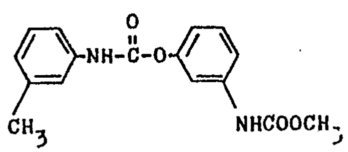 | 0,5 | 0 | 30 | 85 | 100 | 100 | 63 | 47 | 95 | 98 |
| | 1,0 | 6 | 30 | 90 | 100 | 100 | 63 | 67 | 97 | 98 |
| bekannt | 2.0 | 10 | 40 | 100 | 100 | 100 | 63 | 77 | 99 | 98 |

0=keine Schädigung 100=totale Schädigung

Tabelle 4—
Wirkung neuer Verbindungen bei der selektiven Beseitigung von Unkräutern in Ölsaaten bei Nachflaufanwendung im Gewächshaus

| Wirkstoff Nr. | kg/ha | Testpflanzen und % Schädigung Arach. hyp. | Carth. tinct. | Glyc. max | Amar. ret. | Desmod. tort. | Euphorb. genic. | Sesb. exal. | Solan. nigr. | Xanth pens. |
|---|---|---|---|---|---|---|---|---|---|---|
| 1 | 0,5 | 0 | — | 4 | 88 | 98 | 57 | 97 | 50 | 100 |
| | 1,0 | 0 | 0 | 7 | 90 | 98 | 57 | 98 | 60 | 100 |
| | 2,0 | 5 | 0 | 11 | 98 | 100 | 67 | 100 | 60 | 100 |
| 98 | 0,5 | 0 | — | 0 | 100 | 100 | 90 | 100 | 50 | 100 |
| | 1,0 | 0 | 0 | 15 | 100 | 100 | 95 | 100 | 70 | 100 |
| | 2,0 | 10 | 0 | 30 | 100 | 100 | 95 | 100 | 100 | 100 |
| 105 | 0,5 | 0 | — | 0 | 100 | 10 | 100 | 100 | 100 | 40 |
| | 1,0 | 0 | 0 | 0 | 100 | 30 | 100 | 100 | 100 | 40 |
| | 2,0 | 10 | 0 | 0 | 100 | 100 | 100 | 100 | 100 | 40 |
| 8 | 0,5 | 0 | — | 0 | 100 | 10 | 50 | 100 | 40 | 30 |
| | 1,0 | 0 | 0 | 0 | 100 | 10 | 100 | 100 | 50 | 30 |
| | 2,0 | 0 | 0 | 10 | 100 | 60 | 100 | 100 | 50 | 30 |
| 107 | 2,0 | 0 | 0 | 20 | 100 | 20 | 90 | 100 | 60 | 40 |

Tabelle 4 - Fortsetzung

Wirkung neuer Verbindungen bei der selektiven Beseitigung von Unkräutern in Ölsaaten bei Nachflaufanwendung im Gewächshaus

| Wirkstoff Nr. | kg/ha | Arach. hyp. | Carth. tinct. | Glyc. max | Testpflanzen und % Schädigung Amar. ret. | Desmod. tort. | Euphorb. genic. | Sesb. exal. | Solan. nigr. | Xanth pens. |
|---|---|---|---|---|---|---|---|---|---|---|
| 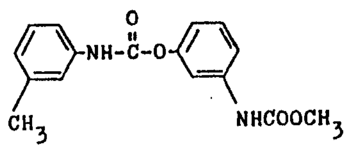 | 0,5 | 0 | — | 30 | 50 | 100 | 100 | 100 | 40 | 30 |
| | 1,0 | 10 | — | 30 | 60 | 100 | 100 | 100 | 60 | 100 |
| bekannt | 2,0 | 10 | — | 35 | 60 | 100 | 100 | 100 | 60 | 100 |

0=keine Schädigung 100=totale Schädigung

Tabelle 5—
Selektive Unkrautbekämpfung mit neuen Verbindungen in Getriede bei Nachauflaufanwendung im Gewächshaus

| Wirkstoff Nr. | kg/ha | Testpflanzen und % Schädigung<br>Triticum aestivum | Mercurialis annua | Sinapis alba | Stellaria media |
|---|---|---|---|---|---|
| 9 | 1,0 | — | 100 | 100 | 100 |
| | 2,0 | 0 | 100 | 100 | 100 |
| 14 | 1,0 | — | — | 100 | 100 |
| | 2,0 | 0 | — | 100 | 100 |
| 17 | 1,0 | — | 100 | 100 | 100 |
| | 2,0 | 0 | 100 | 100 | 100 |
| 37 | 1,0 | — | 0 | 100 | 100 |
| | 2,0 | 0 | 0 | 100 | 100 |
| 35 | 1,0 | — | 0 | 100 | 100 |
| | 2,0 | 0 | 0 | 100 | 100 |
| 39 | 1,0 | — | 0 | 100 | 100 |
| | 2,0 | 0 | 80 | 100 | 100 |
| 45 | 1,0 | — | 40 | 70 | 100 |
| | 2,0 | 0 | 80 | 90 | 100 |
| 85 | 1,0 | — | 100 | 100 | 100 |
| | 2,0 | 0 | 100 | 100 | — |
| 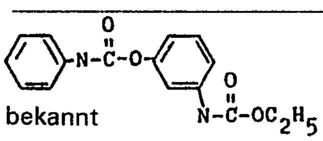 bekannt | 2,0 | 20 | 100 | 100 | 100 |
| 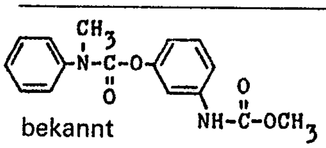 bekannt | 1,0 | 82 | — | 100 | — |
| | 2,0 | 92 | — | 100 | — |

0=keine Schädigung 100=totale Schädigung

Tabelle 5—Fortsetzung

| Wirkstoff Nr. | kg/ha | Triticum aestivum | Testpflanzen und % Schädigung Mercurialis annua | Sinapis alba | Stellaria media |
|---|---|---|---|---|---|
| 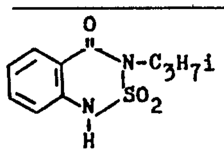 | 1,0 | 0 | 38 | 100 | 100 |
| bekannt | 2,0 | 0 | 55 | 100 | 100 |
| 32 | 2,0 | 0 | — | 100 | 100 |
| 225 | 2,0 | 30 | 70 | 100 | 100 |
| 206 | 0,5 | — | 100 | 100 | 100 |
| | 2,0 | 0 | 100 | 100 | 100 |
| 231 | 0,5 | — | 70 | 100 | 100 |
| | 2,0 | 0 | 100 | 100 | 100 |
| 233 | 0,5 | — | — | 100 | 100 |
| | 2,0 | 0 | — | 100 | 100 |
| 144 | 0,5 | 0 | 100 | — | 100 |
| | 2,0 | 0 | 100 | — | 100 |
| 241 | 0,5 | 0 | 100 | — | 100 |
| | 2,0 | 0 | 100 | — | 100 |
| 217 | 0,5 | 0 | 30 | 70 | — |
| | 2,0 | 0 | 100 | 95 | — |

0=keine Schädigung 100=Pflanzen abgestorben

Tabelle 6 —
Bekämpfung breitblättriger Unkräuter in Erdnüssen bei Nachauflaufanwendung in Gewächshaus

| Wirkstoff Nr. | kg/ha | Testpflanzen und % Schädigung<br>Arachys hypog. | Amaranthus retro. | Desmodium tort. | Sesbania exalt. | Xanthium pensyl. |
|---|---|---|---|---|---|---|
| 90 | 0,5 | 5 | 100 | 100 | 100 | 50 |
| | 2,0 | 20 | 100 | 100 | 100 | 50 |
| 103 | 0,5 | 0 | 100 | 90 | 95 | 50 |
| | 2,0 | 0 | 100 | 90 | 95 | 50 |
| 9 | 0,5 | 10 | 100 | 90 | 95 | 80 |
| | 2,0 | 10 | 100 | 90 | 95 | 90 |
| 14 | 0,5 | 0 | 20 | 20 | 80 | 0 |
| | 2,0 | 0 | 50 | 90 | 95 | 20 |
| 17 | 0,5 | 0 | 10 | 90 | 85 | 0 |
| | 2.0 | 0 | 30 | 90 | 85 | 25 |
| 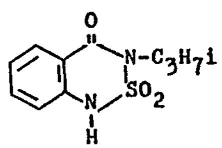 bekannt | 0,5 | 0 | 0 | 0 | 0 | 60 |
| | 2,0 | 10 | 40 | 0 | 20 | 100 |
| 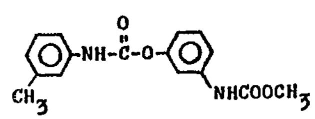 bekannt | 0,5 | 0 | 0 | 100 | 100 | 0 |
| | 2,0 | 0 | 0 | 100 | 100 | 20 |

0=keine Schädigung 100=totale Schädigung

Tabelle 7—
Wirkung neuer Verbindungen bei Nachauflaufanwendung im Gewächshaus

| Wirkstoff Nr. | kg/ha a.S. | Testpflanzen und % Schädigung<br>Centaurea cyanus | Lamium spp. | Sinapis alba | Stellaria media |
|---|---|---|---|---|---|
| 61 | 2,0 | 100 | 100 | 100 | 100 |
| 53 | 2,0 | 100 | 100 | 100 | 100 |
| 58 | 2,0 | 100 | 100 | 100 | 100 |
| 214 | 1,0 | 90 | — | 100 | 100 |
| 239 | 1,0 | 45 | — | 100 | 100 |
| 80 | 1,0 | 90 | — | 100 | 100 |
| 237 | 0,5 | 85 | — | 95 | 100 |
| 236 | 0,5 | 95 | — | 95 | 100 |
| 211 | 1,0 | 85 | — | 100 | 100 |
| 210 | 2,0 | 55 | — | 70 | 100 |
| 216 | 2,0 | 90 | — | 95 | 100 |
| 212 | 1,0 | 55 | — | 100 | 100 |

0=keine Schädigung 100=Pflanzen abgestorben

Tabelle 8—
Neue Verbindungen mit selektiver herbizider Wirkung bei Erdnüssen bei Nachauflaufbehandlung im Gewächshaus

| Wirkstoff Nr. | kg/ha | Testpflanzen und % Schädigung Arach. hyp. | Amar. ret. | Datura stram. | Desmod. spp. | Echin. c. g. | Ipom. spp. | Sesb. exalt. | Solan. nigr. | Xanth. pens. |
|---|---|---|---|---|---|---|---|---|---|---|
| 59 | 0,5 | 0 | 100 | 100 | — | 80 | 50 | 100 | 100 | 40 |
| | 2,0 | 0 | 100 | 100 | — | 80 | 100 | 100 | 100 | 100 |
| 199 | 0,5 | 0 | 95 | 10 | 100 | 30 | 20 | 100 | 100 | 20 |
| | 2,0 | 5 | 100 | 20 | 100 | 30 | 60 | 100 | 100 | 20 |
| 149 | 0,5 | 0 | 100 | 40 | — | 30 | 0 | 100 | 100 | 10 |
| | 2,0 | 0 | 100 | 100 | — | 40 | 100 | 100 | 100 | 30 |
| 213 | 0,5 | 0 | 100 | 100 | — | 40 | 40 | 100 | 100 | 100 |
| | 2,0 | 0 | 100 | 100 | — | 60 | 80 | 100 | 100 | 100 |
| 181 | 0,5 | 0 | 100 | 100 | — | 10 | 80 | 90 | 100 | 20 |
| | 2,0 | 0 | 100 | 100 | — | 40 | 100 | 100 | 100 | 20 |
| 132 | 0,5 | 0 | 100 | 100 | — | 60 | 10 | 100 | 100 | 100 |
| | 2,0 | 0 | 100 | 100 | — | 60 | 40 | 100 | 100 | 100 |
| 209 | 0,5 | 0 | 45 | 100 | — | 50 | 20 | 100 | 100 | 40 |
| | 2,0 | 15 | 100 | 100 | — | 50 | 40 | 100 | 100 | 40 |
| 67 | 0,25 | 0 | 18 | — | 25 | — | — | 100 | 28 | — |
| | 2,0 | 0 | 100 | — | 100 | — | — | 100 | 65 | — |
| 150 | 1,0 | 0 | 70 | — | 100 | — | 60 | — | — | 80 |

0=keine Schädigung 100=Pflanzen abgetötet

Tabelle 9—
Neue Verbindungen mit selektiver herbizider Wirkung bei Getreide und Mais bei Nachauflaufanwendung im Gewächshaus

| Wirkstoff Nr. | kg/ha | Testpflanzen und % Schädigung<br>Tritic. aest. | Zea mays | Amar. ret. | Cent. cyan. | Chrys. seg. | Lamium spp. | Matric. spp. | Sinapis alba | Solan. nigr. |
|---|---|---|---|---|---|---|---|---|---|---|
| 218 | 0,5 | 0 | 0 | 100 | 100 | 100 | 100 | 90 | 95 | 100 |
| | 1,0 | 0 | 0 | 100 | 100 | 100 | 100 | 100 | 100 | 100 |
| 201 | 0,5 | 0 | 0 | 100 | 100 | 100 | 100 | 50 | 90 | 100 |
| | 1,0 | 0 | 10 | 100 | 100 | 100 | 100 | 100 | 95 | 100 |
| 118 | 0,5 | 0 | 0 | 100 | 30 | — | 70 | 40 | 100 | 10 |
| | 2,0 | 0 | 0 | 100 | 60 | — | 100 | 60 | 100 | 30 |
| 187 | 0,5 | 0 | 0 | 100 | 60 | — | 100 | 40 | 100 | 70 |
| | 2,0 | 20 | 10 | 100 | 60 | — | 100 | 60 | 100 | 100 |
| 115 | 2,0 | 0 | 0 | 73 | — | 93 | 70 | — | 100 | 60 |

0=keine Schädigung 100=Pflanzen abgetötet

Tabelle 10
Neue Wirkstoffe mit selektiver herbizider Wirkung bei Baumwolle und Zuckerrüben bei Nachauflaufbehandlung im Gewächshaus

| Wirkstoff Nr. | kg/ha | Beta vulg. | Gossyp. hirs. | Testpflanzen und % Schädigung<br>Amar. ret. | Datura stram. | Lamium spp. | Solan. nigr. |
|---|---|---|---|---|---|---|---|
| 240 | 0,25 | 0 | 0 | 100 | 100 | 100 | 100 |
| | 2,0 | 0 | 5 | 100 | 100 | 100 | 100 |
| 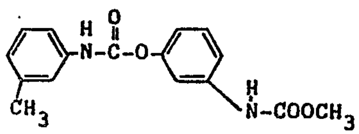 | 0,25 | 0 | 27 | 6 | 82 | 100 | 46 |
| bekannt | 2,0 | 2 | 78 | 16 | 100 | 100 | 92 |

0=keine Schädigung 100=Pflanzen abgestorben

Tabelle 11—
Wirkstoffe mit selektiver herbizider Wirkung bei Mais und Soja bei Nachauflaufanwendung im Gewächshaus

| Wirkstoff Nr. | kg/ha | Glyc. max | Zea mays | Amar. ret. | Testpflanzen und % Schädigung<br>Chenop. album | Desmod. tort. | Ipomoea spp. | Lamium spp. | Mercur. annua | Setaria spp. |
|---|---|---|---|---|---|---|---|---|---|---|
| 70 | 0,5 | 0 | 0 | 100 | 100 | 100 | 80 | 100 | 70 | 55 |
| | 2,0 | 10 | 10 | 100 | 100 | 100 | 90 | 100 | 100 | 70 |
| 151 | 0,25 | 0 | 0 | 30 | 100 | — | 70 | 100 | 60 | 70 |
| | 2,0 | 0 | 5 | 100 | 100 | 100 | 95 | 100 | 100 | 85 |
| 176 | 0,25 | 0 | 0 | 100 | 100 | — | 20 | 95 | 50 | — |
| | 2,0 | 0 | 0 | 100 | 100 | 100 | 90 | 100 | 100 | — |

0=keine Schädigung 100=Pflanzen abgestorben

Tabelle 12
Wirkstoffe mit selektiver herbizider Wirkung bei Baumwolle, Erdnüssen und Reis bei Nachauflaufanwendung im Gewächshaus

| Wirkstoff Nr. | kg/ha | Testpflanzen und % Schädigung Arachys hyp. | Gossyp. hirs. | Oryza sat. | Amar. ret. | Datura stram. | Lamium spp. | Polyg. pers. | Solan. nigr. |
|---|---|---|---|---|---|---|---|---|---|
| 207 | 0,25 | 0 | 0 | 10 | 70 | 100 | 70 | 100 | 100 |
| | 1,0 | 0 | 0 | 10 | 100 | 100 | 100 | 100 | 100 |
| | 2,0 | 0 | 10 | 10 | 100 | 100 | 100 | 100 | 100 |
| 250 | 1,0 | 0 | 5 | 5 | 45 | 100 | 100 | 80 | 100 |
| | 2,0 | 0 | 5 | 5 | 100 | 100 | 100 | 90 | 100 |
| 223 | 0,25 | 10 | 10 | 0 | 45 | 75 | 100 | 70 | 100 |
| | 1,0 | 10 | 20 | 0 | 100 | 100 | 100 | 100 | 100 |

0=keine Schädigung 100=Pflanzen abgestorben

Tabelle 13—
Selektive Bekämpfung von Unkräutern mit neuen Verbindungen in Getreide und Mais bei Nachauflaufanwendung im Gewächshaus

| Wirkstoff Nr. | kg/ha a.S. | Testpflanzen und % Schädigung | | | | |
|---|---|---|---|---|---|---|
| | | Triticum aestivum | Zea mays | Lamium amplexicaule | Sinapis alba | Stellaria media |
| 222 | 0,25 | 10 | 0 | 100 | 95 | 95 |
| 235 | 1,0 | 10 | 15 | 100 | 90 | 98 |
| 111 | 2,0 | 0 | 10 | 100 | — | 90 |
| 150 | 1,0 | 0 | 15 | 80 | 100 | 100 |
| 134 | 0,5 | 0 | 15 | 100 | 90 | 100 |
| 248 | 1,0 | 10 | 10 | 100 | — | 90 |

0=keine Schädigung 100=Pflanzen völlig abgestorben

Tabelle 14—
Bekämpfung von Unkräutern mit neuen Verbindungen in Erdnüssen bei Nachauflaufanwendung im Gewächshaus

| Wirkstoff-Nr. | kg/ha a.S. | Testpflanzen und % Schädigung<br>Arachis hypogaea | Amaranthus retroflexus | Nicandra physaloides | Sesbania exaltata | Solanum nigrum |
|---|---|---|---|---|---|---|
| 220 | 0,5 | 0 | 100 | 100 | 100 | 100 |
| 208 | 0,5 | 0 | 100 | 100 | 100 | 100 |
| 232 | 1,0 | 5 | 100 | — | 100 | 100 |

0=keine Schädigung 100=Pflanzen völlig abgestorben

Tabelle 15—
Bekämpfung von Euphorbia geniculata mit neuen Verbindungen in Sojabohnen bei Nachauflaufanwendung im Gewächshaus

| Wirkstoff-Nr. | kg/ha a.S. | Testpflanzen und % Schädigung<br>Glycine max | Euphorbia geniculata |
|---|---|---|---|
| 87 | 1,0 | 0 | 90 |
| | 2,0 | 0 | 90 |
| 235 | 1,0 | 15 | 98 |
| 222 | 1,0 | 15 | 80 |
| 221 | 1,0 | 15 | 80 |

0=keine Schädigung 100=Pflanzen völlig abgestorben

Tabelle 16—
Vernichtung von Chenopodium alkbum und Stellaria media mit neuen Verbindungen in Rüben bei Nachauflaufanwendung im Gewächshaus

| Wirkstoff-Nr. | kg/ha a.S. | Testpflanzen und % Schädigung<br>Beta vulgaris | Chenopodium album | Stellaria media |
|---|---|---|---|---|
| 251 | 0,5 | 10 | 94 | 98 |

0=keine Schädigung 100=Pflanzen völlig abgestorben

Tabelle 17—
Bekämpfung von Chenopodium album und Sesbania exaltata mit neuen Verbindungen in Pfefferminze bei Nachauflaufanwendung im Gewächshaus

| Wirkstoff-Nr. | kg/ha a.S. | Testpflanzen und % Schädigung<br>Mentha piperita | Chenopodium album | Sesbania exaltata |
|---|---|---|---|---|
| 215 | 1,0 | 0 | 95 | 90 |
| 258 | 1,0 | 0 | 100 | 80 |

0=keine Schädigung 100=Pflanzen völlig abgestorben

Tabelle 18—
Herbizide Wirkung bei Nachauflauf im Gewächshaus

| Wirkstoff-Nr. | kg/ha a.S. | Testpflanzen und % Schädigung<br>Centaurea cyanus | Ipomoea spp. | Echinochloa crus galli |
|---|---|---|---|---|
| 246 | 3,0 | 100 | 100 | 100 |
| 215 | 3,0 | 100 | 100 | 90 |
| 252 | 3,0 | 90 | 70 | — |
| 258 | 3,0 | 100 | 100 | 100 |
| 152 | 3,0 | 100 | 100 | — |

**Patentansprüche**

1. Diurethan der allgemeinen Formel

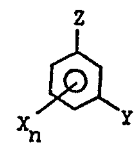

in der Z den Rest

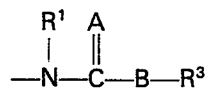

und Y den Rest

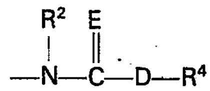

bedeutet, wobei Z immer verschieden ist von Y und $R^1$ und $R^2$ jeweils unabhängig voneinander Wasserstoff, Alkyl mit 1 bis 4 Kohlenstoffatomen, Alkoxyalkyl mit 2 bis 4 Kohlenstoffatomen, Alkoxycarbonylalkyl mit 3 bis 5 Kohlenstoffatomen, Halogenalkyl mit 1 bis 4 Kohlenstoffatomen, gegebenenfalls durch Alkyl mit 1 bis 4 Kohlenstoffatomen oder Halogen substituiertes Benzyl, $R^3$ und $R^4$ jeweils unabhängig voneinander unsubstituiertes Alkyl mit 1 bis 6 Kohlenstoffatomen oder durch Halogen oder Alkoxy mit 1 bis 2 Kohlenstoffatomen oder durch Halogen substituiertes oder unsubstituiertes Phenyl substituiertes Alkyl mit 1 bis 4 Kohlenstoffatomen, Alkenyl mit 2 bis 4 Kohlenstoffatomen, Alkinyl mit 3 bis 4 Kohlenstoffatomen, gegebenenfalls durch Alkyl mit 1 bis 4 Kohlenstoffatomen, substituiertes Cycloalkyl mit 5 bis 8 Kohlenstoffatomen, Bicycloalkyl mit 7 bis 8 Kohlenstoffatomen, Tricycloalkyl mit 10 bis 15 Kohlenstoffatomen; einen Phenylring mit ankondensiertem Ringsystem, phenyl, ein- oder mehrfach substituiertes Phenyl, mit den Substituenten Alkyl mit 1 bis 4 Kohlenstoffatomen, Halogen oder Alkoxy mit 1 bis 3 Kohlenstoffatomen bedeuten und X Wasserstoff, Alkyl mit 1 bis 4 Kohlenstoffatomen, Halogenalkyl mit 1 bis 3 Kohlenstoffatomen, Alkoxy mit 1 bis 2 Kohlenstoffatomen, Halogen, Nitro oder Amino, n 1 bis 4 und A, B, D, E jeweils unabhängig voneinander Sauerstoff oder Schwefel bedeuten, wobei nicht alle Reste A, B, D, E gleichzeitig Sauerstoff bedeuten und wobei mindestens einer dieser Reste immer Schwefel bedeutet.

2. Herbizid, enthaltend als Wirkstoff ein Diurethan gemäß Anspruch 1.

3. Verfahren zur Bekämpfung unerwünschten Pflanzenwuchses durch Behandlung mit einem Diurethan, *dadurch gekennzeichnet*, daß man ein Diurethan gemäß Anspruch 1 verwendet.

4. Verfahren zur Herstellung eines Diurethans gemäß Anspruch 1, *dadurch gekennzeichnet*, daß man ein Phenylaminourethan der Formel

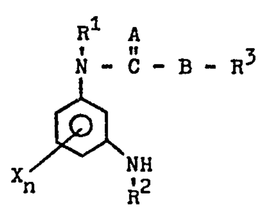

in der $R^1$, $R^2$, $R^3$, A, B, X und n die in Anspruch 1 genannte Bedeutung haben, in an sich bekannter Weise umsetzt mit einem Halogenameisensäureester der Formel

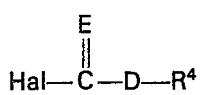

in der $R^4$, D und E die in Anspruch 1 genannte Bedeutungen haben und Hal ein Halogenatom bedeutet oder mit Schwefelkohlenstoff und einem Alkylierungsmittel bei einer Temperatur im Bereich von −20° bis 150°C umsetzt.

5. Diurethan gemäß Anspruch 1 mit der Formel

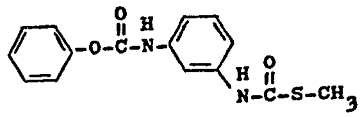

6. Diurethan gemäß Anspruch 1 mit der Formel

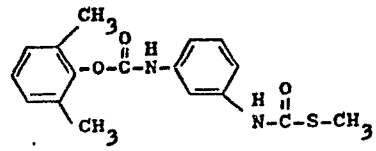

7. Diurethan gemäß Anspruch 1 mit der Formel

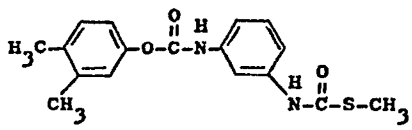

8. Diurethan gemäß Anspruch 1 mit der Formel

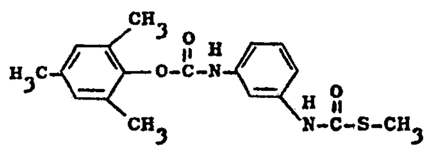

9. Diurethan gemäß Anspruch 1 mit der Formel

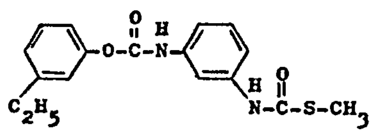

10. Diurethan gemäß Anspruch 1 mit der Formel

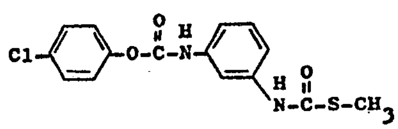

11. Diurethan gemäß Anspruch 1 mit der Formel

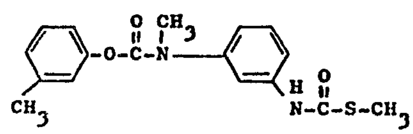

12. Diurethan gemäß Anspruch 1 mit der Formel

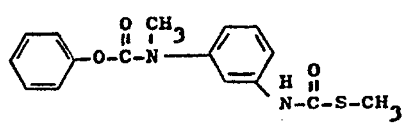

13. Diurethan gemäß Anspruch 1 mit der Formel

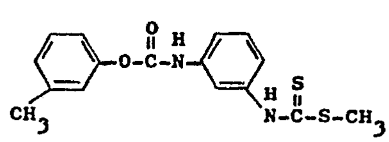

14. Diurethan gemäß Anspruch 1 mit der Formel

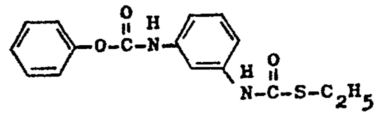

15. Diurethan gemäß Anspruch 1 mit der Formel

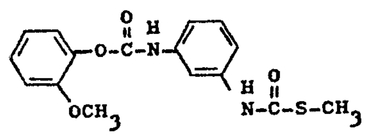

16. Diurethan gemäß Anspruch 1 mit der Formel

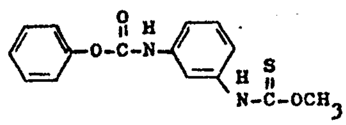

17. Diurethan gemäß Anspruch 1 mit der Formel

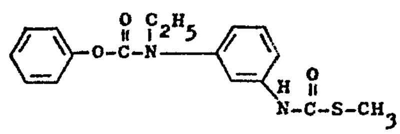

18. Diurethan gemäß Anspruch 1 mit der Formel

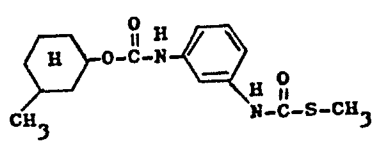

## Claims

1. A diurethane of the general formula

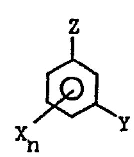

where Z denotes the radical

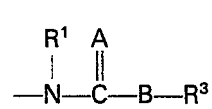

and Y denotes the radical

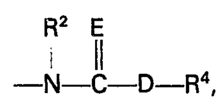

Z always being different from Y and $R^1$ and $R^2$ being identical or different and each denoting hydrogen, alkyl of 1 to 4 carbon atoms, alkoxyalkyl of 2 to 4 carbon atoms, alkoxycarbonylalkyl of 3 to 5 carbon atoms, haloalkyl of 1 to 4 carbon atoms, unsubstituted benzyl, or benzyl substituted by alkyl of 1 to 4 carbon atoms or halogen, $R^3$ and $R^4$ being identical or different and each denoting unsubstituted alkyl of 1 to 6 carbon atoms, alkyl of 1 to 4 carbon atoms

substituted by halogen, alkoxy of 1 or 2 carbon atoms, halogen-substituted phenyl or unsubstituted phenyl, alkenyl of 2 to 4 carbon atoms, alkynyl of 3 or 4 carbon atoms, unsubstituted or $C_1$—$C_4$-alkyl-substituted cycloalkyl of 5 to 8 carbon atoms, bicycloalkyl of 7 or 8 carbon atoms, tricycloalkyl of 10 to 15 carbon atoms, phenyl with a fused ring system, phenyl or mono- or poly-substituted phenyl with the substituents alkyl of 1 to 4 carbon atoms, halogen or alkoxy of 1 to 3 carbon atoms, X denotes hydrogen, alkyl of 1 to 4 carbon atoms, haloalkyl of 1 to 3 carbon atoms, alkoxy of 1 or 2 carbon atoms, halogen, nitro or amino, n denotes one of the integers 1, 2, 3 and 4, and A, B, D and E are identical or different and each denotes oxygen or sulfur (with the proviso that the radicals A, B, D and E are not simultaneously oxygen and one of the radicals always denotes sulfur).

2. A herbicide containing as the active ingredient a diurethane as claimed in claim 1.

3. A process for combatting the growth of unwanted plants by treatment with a diurethane, wherein a diurethane as claimed in claim 1 is used.

4. A process for the manufacture of a diurethane as claimed in claim 1, wherein a phenylaminourethane of the formula

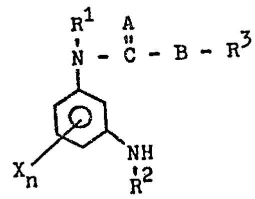

where $R^1$, $R^2$, $R^3$, A, B, X and n have the meanings given in claim 1, is reacted in a conventional manner with a haloformic acid ester of the formula

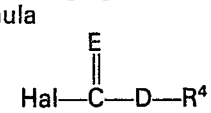

where $R^4$, D and E have the meanings given in claim 1 and Hal is halogen, or is reacted with carbon disulfide and an alkylating agent at from −20° to 150°C.

5. A diurethane as claimed in claim 1 of the formula

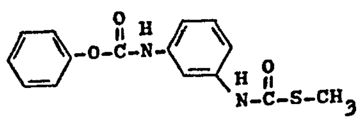

6. A diurethane as claimed in claim 1 of the formula

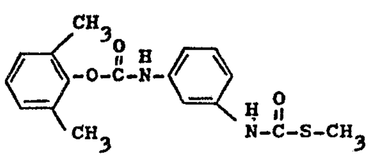

7. A diurethane as claimed in claim 1 of the formula

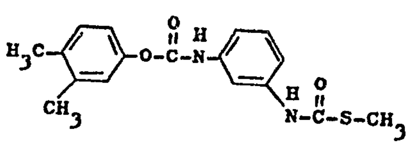

8. A diurethane as claimed in claim 1 of the formula

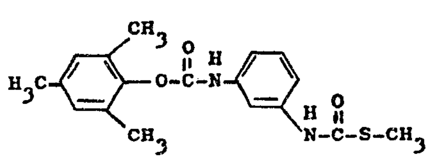

9. A diurethane as claimed in claim 1 of the formula

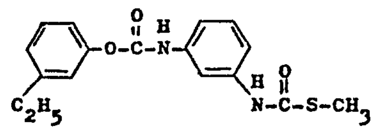

10. A diurethane as claimed in claim 1 of the formula

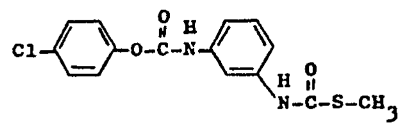

11. A diurethane as claimed in claim 1 of the formula

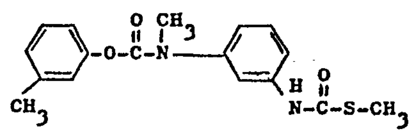

12. A diurethane as claimed in claim 1 of the formula

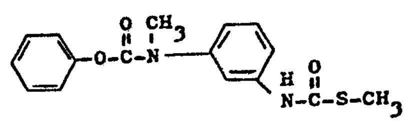

13. A diurethane as claimed in claim 1 of the formula

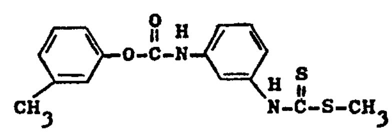

14. A diurethane as claimed in claim 1 of the formula

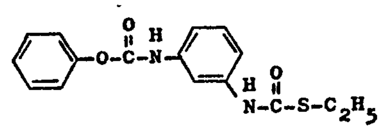

15. A diurethane as claimed in claim 1 of the formula

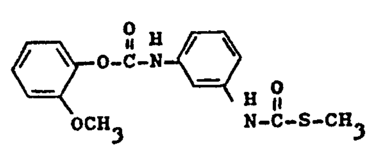

16. A diurethane as claimed in claim 1 of the formula

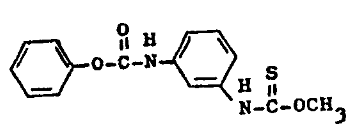

17. A diurethane as claimed in claim 1 of the formula

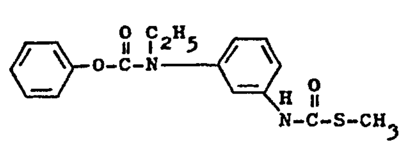

18. A diurethane as claimed in claim 1 of the formula

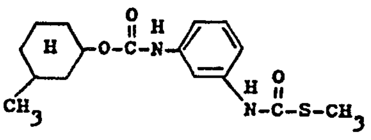

## Revendications

1. Diuréthane de formule générale

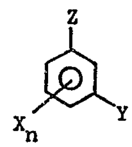

dans laquelle Z représente le reste

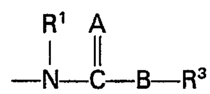

et Y le reste

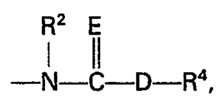

Z étant toujours différent de Y et $R^1$ et $R^2$ représentant indépendamment l'un de l'autre de l'hydrogène, un alkyle en $C_1$ à $C_4$, un alcoxyalkyle en $C_2$ à $C_4$, un alkoxycarbonylalkyle en $C_3$ à $C_5$, un halogène-alkyle en $C_1$ à $C_4$, un benzyle éventuellement substitué par un alkyle en $C_1$ à $C_4$ ou un halogène, $R^3$ et $R^4$ représentant indépendamment l'un de l'autre un alkyle non substitué en $C_1$ à $C_6$ ou un alkyle en $C_1$ à $C_4$ substitué par un halogène ou un alcoxy en $C_1$ à $C_2$ ou par un phényle substitué par un halogène ou non substitué, un alcényle en $C_2$ à $C_4$, un alcinyle en $C_3$ à $C_4$, un cycloalkyle en $C_5$ à $C_8$ éventuellement substitué par un alkyle en $C_1$ à $C_4$, un bicycloalkyle en $C_7$ à $C_8$, un tricycloalkyle en $C_{10}$ à $C_{15}$, un noyau phényle sur lequel est condensé un cycle, phényle, phényle substitué une ou plusieurs fois avec comme substituants un alkyle en $C_1$ à $C_4$, un halogène ou un alcoxy en $C_1$ à $C_3$, et X représente de l'hydrogène, un alkyle en $C_1$ à $C_4$, un halogène-alkyle en $C_1$ à $C_3$, un alcoxy en $C_1$ à $C_2$, un halogène, un nitro ou un amino, n vaut de 1 à 4 et A, B, D, E représentent chacun indépendamment l'un de l'autre de l'oxygène ou du soufre, les restes A, B, D, E ne représentant pas tous de l'oxygène, l'un au moins de ces restes étant du soufre.

2. Herbicide contenant comme principe actif un diuréthane selon la revendication 1.

3. Procédé de lutte contre la croissance des plantes adventices par traitement avec un diuréthane, caractérisé par le fait qu'on utilise un diuréthane selon la revendication 1.

4. Procédé de préparation d'un diuréthane selon la revendication 1, caractérisé par le fait qu'on fait réagir, de façon connue en soi, un phénylamino-uréthane de formule

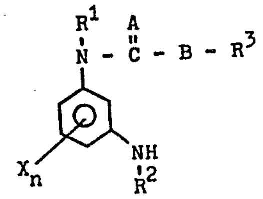

dans laquelle $R^1$, $R^2$, $R^3$, A, B, X et n ont la signification indiquée dans la revendication 1, avec un ester d'acide halogénoformique de formule

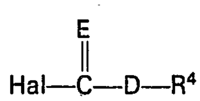

dans laquelle $R^4$, D et E ont la signification indiquée dans la revendication 1 et Hal est un atome

d'halogène, ou avec du sulfure de carbone et un agent d'alkylation à une température comprise entre −20 et 150°C.

5. Diuréthane selon la revendication 1 de formule

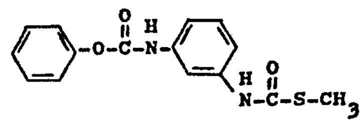

6. Diuréthane selon la revendication 1 de formule

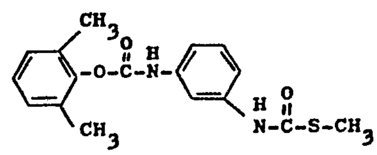

7. Diuréthane selon la revendication 1 de formule

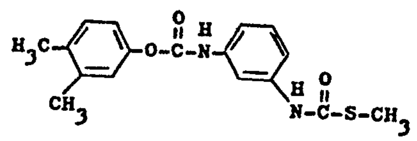

8. Diuréthane selon la revendication 1 de formule

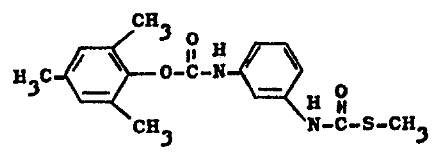

9. Diuréthane selon la revendication 1 de formule

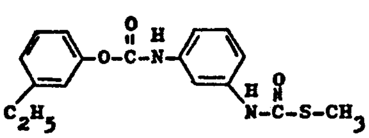

10. Diuréthane selon la revendication 1 de formule

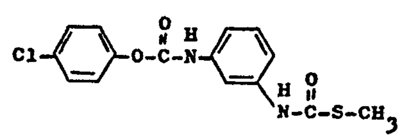

11. Diuréthane selon la revendication 1 de formule

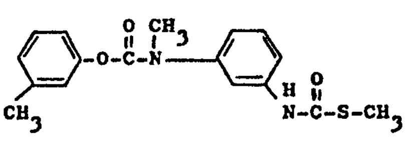

12. Diuréthane selon la revendication 1 de formule

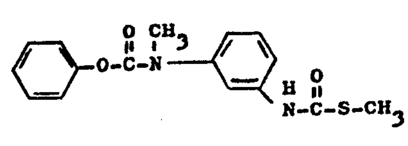

13. Diuréthane selon la revendication 1 de formule

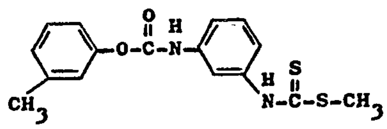

14. Diuréthane selon la revendication 1 de formule

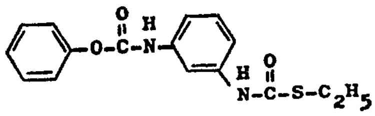

15. Diuréthane selon la revendication 1 de formule

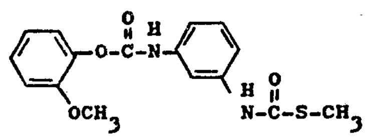

16. Diuréthane selon la revendication 1 de formule

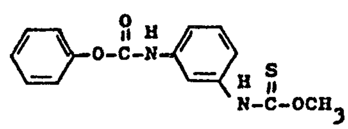

17. Diuréthane selon la revendication 1 de formule

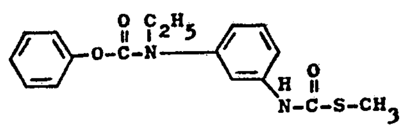

18. Diuréthane selon la revendication 1 de formule